# EUROPEAN PATENT SPECIFICATION

(11) **EP 1 864 624 B1**
(45) Date of publication and mention of the grant of the patent: **01.04.2015**
(21) Application number: 06714316.4
(22) Date of filing: 22.02.2006
(51) Int. Cl.: A61B 19/00, A61B 18/14

(54) **SURGERY ASSISTING APPARATUS**
OPERATIONSUNTERSTÜTZUNGSGERÄT
APPAREIL D'ASSISTANCE A LA CHIRURGIE

(30) Priority: 31.03.2005 JP 2005104125; 31.03.2005 JP 2005104129
(43) Date of publication of application: 12.12.2007
(73) Proprietor: OLYMPUS MEDICAL SYSTEMS CORP., Tokyo 151-0072 (JP)
(72) Inventor: ONODA, Fumiyuki, c/o Olympus Medical Systems Corp., Tokyo 151-0072 (JP); NIWA, Hiroshi c/o Olympus Medical Systems Corp., Tokyo 151-0072 (JP); SATO, Minoru c/o Olympus Medical Systems Corp., Tokyo 151-0072 (JP); ODA, Tomohiko c/o Olympus Medical Systems Corp., Tokyo 151-0072 (JP); MIYOSHI, Yoshitaka c/o Olympus Medical Systems Corp., Tokyo 151-0072 (JP); MIYAKE, Kensuke c/o Olympus Medical Systems Corp., Tokyo 151-0072 (JP); AIZAWA, Chieko c/o Olympus Medical Systems Corp,, Tokyo 151-0072 (JP)
(74) Representative: von Hellfeld, Axel
(86) International application number: PCT/JP2006/303176
(87) International publication number: WO 2006/112136

(56) References cited:
- EP-A1- 1 374 791
- WO-A1-02/39917
- WO-A1-97/29709
- JP-A- 07 255 744
- JP-A- 2001 061 776
- JP-A- 2002 253 480
- JP-A- 2002 253 480
- JP-B2- 3 634 416
- US-A1- 2003 078 509
- Jennifer R. Cook ET AL: "Laparovaginal hysterectomy: A decade of evolution", The Australian and New Zealand Journal of Obstetrics and Gynaecology, vol. 44, no. 2, 1 April 2004 (2004-04-01), pages 111-116, XP055099675, ISSN: 0004-8666, DOI: 10.1111/j.1479-828X.2004.00170.x

## Description

### Technical Field

The present invention relates to a surgery assisting apparatus and a treatment assisting apparatus which assist a surgery using a magnetic-field generating element and a magnetic-field detecting element.

### Background Art

In recent years, there has been used an endoscope shape detecting apparatus which detects a shape and the like of an endoscope inserted, for example, into a body cavity using a magnetic-field generating element and a magnetic-field detecting element, and displays the detected shape by display means.

For example, Japanese Unexamined Patent Application Publications No. 2003-245243 and No. 2003-290129 disclose an apparatus which detects the shape of an endoscope using magnetic fields, and displays the detected shape of the endoscope. In these conventional examples, a plurality of magnetic-field generating elements disposed at a predetermined interval in an insertion portion of the endoscope which is inserted in a body are driven to generate magnetic fields therearound, and three-dimensional positions of the respective magnetic-field generating elements are detected by magnetic-field detecting elements disposed outside the body. Then, a curve continuously linking the respective magnetic-field generating elements is generated, and a three-dimensional image representing a model of the insertion portion is displayed by the display means.

An operator and the like can have a grasp of the position of a distal end portion of the insertion portion inserted in a body, insertion shape, and the like by observing the image. This helps the operator smoothly perform the work of inserting the insertion portion into a target region, for example.

Meanwhile, in a surgical operation, a high-frequency cauterizing apparatus, ultrasonic treatment apparatus, and the like are used when performing treatment on a diseased organ.

However, in the vicinity of the region to be treated of the diseased organ, luminal organs which are irrelevant of the diseased organ, such as blood vessels, urinary tract, and the like, are spread. In a surgical operation, it is necessary to perform treatment avoiding the luminal organs when treating the diseased organ with a high-frequency cauterizing apparatus. However, the luminal organs are often hidden by the diseased organ, so that there are problems that visual confirm of the luminal organs is difficult and procedures can not be smoothly performed.

In addition, in an inspection using an endoscope, treatment instruments such as a biopsy forceps and clip are used by insertion into a forceps channel in order to biopsy tissues or to perform various treatments such as arrest of hemorrhage on the tissues. However, treatment has been conventionally performed while merely observing an endoscope image on the monitor and the like, so that there has been a problem that the region of the treated tissue can be confirmed only by an observation image.

Therefore, it is difficult to objectively judge whether or not the treatment has been appropriately performed after the inspection unless the observation image at the time of the treatment is frozen to be recorded, so that it is necessary to manually record the images before and after the treatment. As a result, inspection has been troublesome.

Furthermore, the treatment instrument such as a clip is sometimes detained in a body after the inspection or treatment. However, the detained state of the clip conventionally could be confirmed only by an X-ray transmission image or an endoscope observation image.

The present invention is achieved in view of above circumstances, and an object of the present invention is to provide a surgery assisting apparatus capable of easily and surely detecting luminal organs irrelevant of treatment and assisting smooth execution of procedures.

JP 2002 253480 A discloses the preamble of claim 1, in particular a medical treatment assisting device comprising a probe, forceps and at least one sensor for detecting positions in each of the probe and the forceps. Each sensor for detecting positions detects the magnetic field generated by an exterior magnetic field generator and an arithmetic unit computes the three-dimensional coordinates position of each sensor for detecting positions.

### Disclosure of Invention

### Means for Solving the Problem

A surgery assisting apparatus according to claim 1 is provided.

### Brief Description of the Drawings

FIG. 1 is a configurational view showing a configuration of a surgery system according to a first example.
FIG. 2 is a view showing a configuration of a probe of FIG. 1.
FIG 3 is a view showing a configuration of a surgical tool of FIG. 1.
FIG 4 is a view showing a disposition example of coils incorporated in a coil unit of FIG 1.
FIG. 5 is a configurational view showing a configuration of a luminal organ shape detecting apparatus of FIG. 1.
FIG. 6 is a view showing configurations of a reception block and a control block of FIG. 5.
FIG. 7 is a view showing a detailed configuration of the reception block of FIG. 5.
FIG. 8 is a timing view showing a working of a two-port memory and the like of FIG 6.
FIG. 9 is a flowchart describing an action of the luminal organ shape detecting apparatus of FIG. 1.
FIG. 10 is an explanatory view describing processings of FIG 9.
FIG. 11 is a view showing a configuration of a first modification example of a probe of FIG. 1.
FIG 12 is a view showing a configuration of a second modification example of the probe of FIG. 1.
FIG. 13 is a view showing a configuration of a surgical tool according to a second example.
FIG. 14 is a flowchart describing an action of the luminal organ shape detecting apparatus when using the surgical tool of FIG. 13.
FIG. 15 is a first explanatory view describing processings of FIG. 14.
FIG. 16 is a second explanatory view describing the processings of FIG. 14.
FIG. 17 is a third explanatory view describing the processings of FIG 14.
FIG. 18 is a configurational view showing a configuration of a surgery system according to an embodiment of the present invention.
FIG. 19 is a flowchart describing an action of a luminal organ shape detecting apparatus of FIG. 18.
FIG. 20 is a configurational view showing a configuration of a surgery system according to a third example.
FIG. 21 is a flowchart describing an action of a luminal organ shape detecting apparatus of FIG. 20.
FIG. 22 is an explanatory view describing processings of FIG. 21.
FIG 23 is a configurational view showing a configuration of a surgery system according to a fourth example.
FIG. 24 is an explanatory view describing an action of a luminal organ shape detecting apparatus of FIG. 23.
FIG. 25 is a configurational view showing a configuration of a surgery system according to a fifth example.
FIG. 26 is an explanatory view describing an action of a luminal organ shape detecting apparatus of FIG. 25.
FIG. 27 is a view showing a configuration of a surgical tool according to a sixth example.
FIG. 28 is a cross-sectional view showing a cross section cut along A-A line of FIG 27.
FIG. 29 is a configurational view showing a configuration of an endoscope system according to a seventh example.
FIG. 30 is a view showing a disposition example of coils incorporated in a coil unit of FIG 29.
FIG. 31 is a configurational view showing a configuration of an endoscope shape detecting apparatus of FIG 29.
FIG 32 is a view showing configurations of a reception block and a control block of FIG. 31.
FIG. 33 is a view showing a detailed configuration of the reception block of FIG. 31.
FIG. 34 is a timing view showing a working of a two-port memory and the like of FIG 32.
FIG. 35 is a view showing a configuration of an electronic endoscope of FIG. 29.
FIG 36 is a first view showing a configuration of a biopsy forceps as a treatment instrument of FIG 29.
FIG. 37 is a second view showing a configuration of the biopsy forceps of FIG. 29.
FIG. 38 is a view showing a configuration of a first modification example of the biopsy forceps of FIG. 37.
FIG. 39 is a flowchart describing an action of the endoscope shape detecting apparatus of FIG 29.
FIG. 40 is a first view describing processings of FIG. 39.
FIG. 41 is a second view describing the processings of FIG. 39.
FIG. 42 is a third view describing the processings of FIG. 39.
FIG. 43 is a fourth view describing the processings of FIG. 39.
FIG. 44 is a flowchart describing a modification example of an action of the endoscope shape detecting apparatus of FIG. 29.
FIG. 45 is a first view describing processings of FIG. 44.
FIG. 46 is a second view describing the processings of FIG. 44.
FIG 47 is a third view describing the processings of FIG. 44.
FIG. 48 is a fourth view showing the processings of FIG. 44.
FIG. 49 is a view showing a configuration of a second modification example of the biopsy forceps of FIG 37.
FIG 50 is a view showing a configuration of a source coil portion of FIG. 49.
FIG. 51 is a first view showing a first modification example of a treatment instrument of FIG. 29.
FIG 52 is a second view showing the first modification example of the treatment instrument of FIG. 29.
FIG. 53 is a view describing an action of the treatment instrument of FIG. 51.
FIG. 54 is a first view showing a second modification example of the treatment instrument of FIG. 29.
FIG. 55 is a second view showing the second modification example of the treatment instrument of FIG. 29.
FIG. 56 is a view showing a third modification example of the treatment instrument of FIG. 29.
FIG. 57 is a view showing a configuration of a third modification example of the biopsy forceps of FIG. 37.
FIG. 58 is a view showing a configuration of a fourth modification example of the biopsy forceps of FIG 37.

### Best Mode for Carrying Out the Invention

Hereinafter, examples and an embodiment of the present invention will be described with reference to the drawings.

### (First Example)

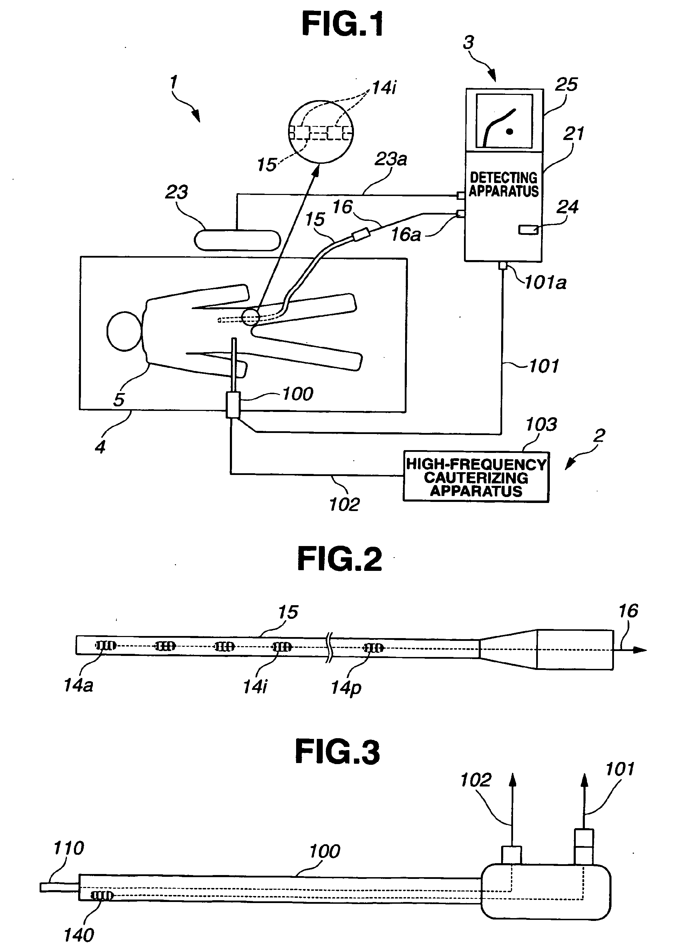

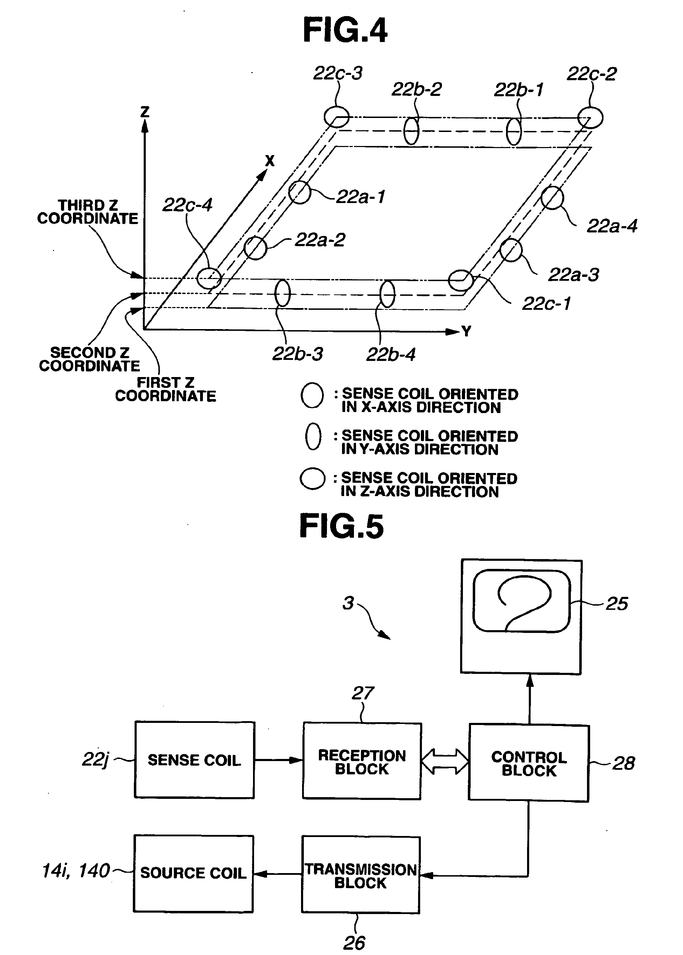

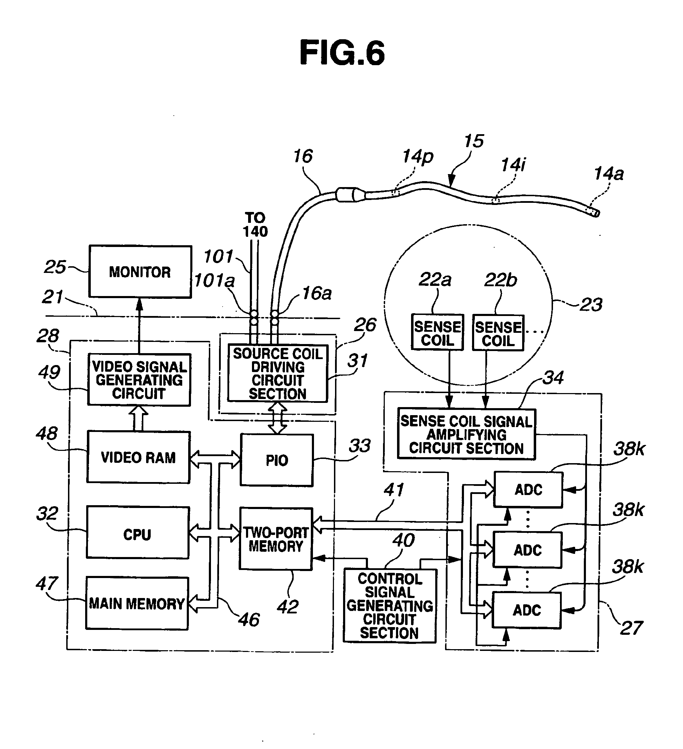

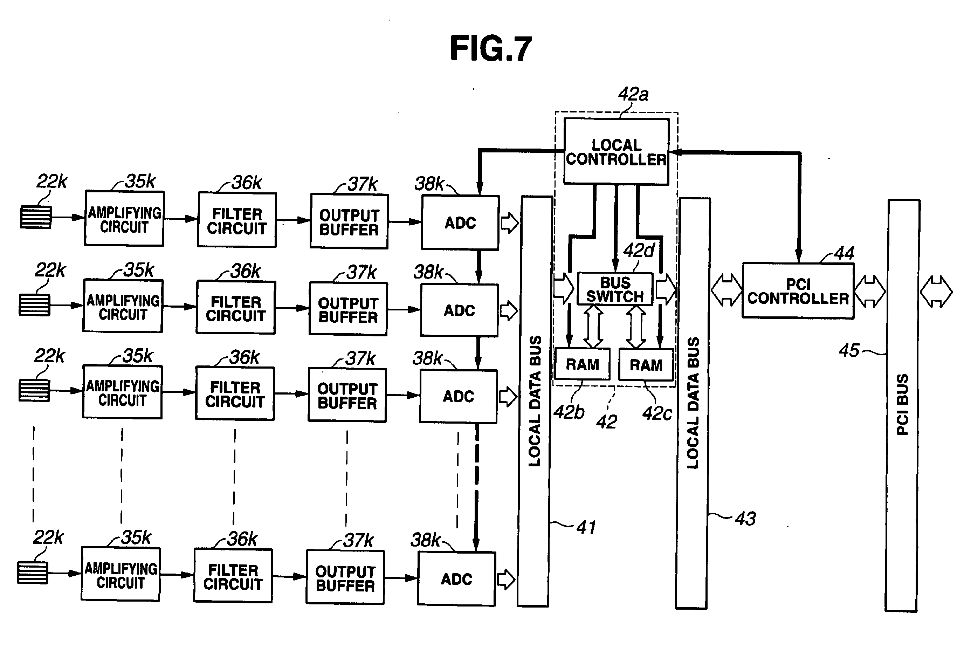

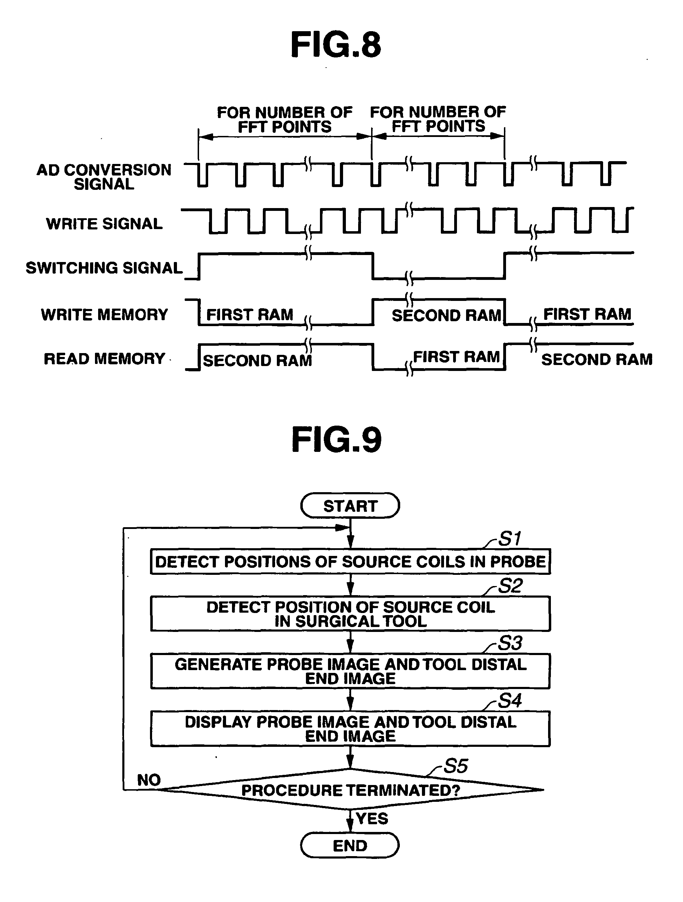

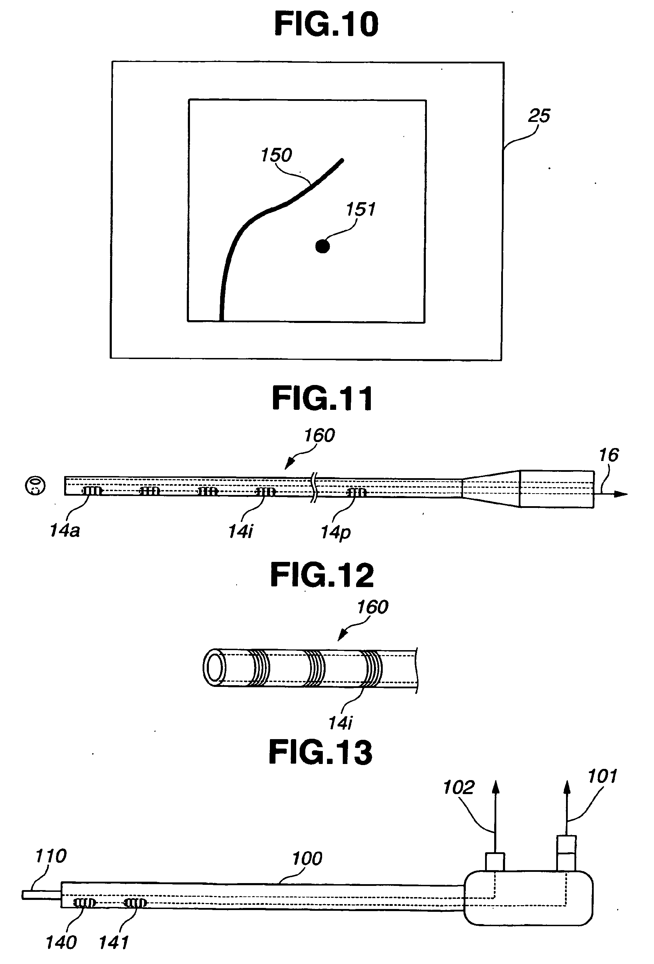

FIGS. 1 to 12 relate to the first example useful for understanding the present invention, in which: FIG. 1 is a configurational view showing a configuration of a surgery system; FIG. 2 is a view showing a configuration of a probe of FIG. 1; FIG. 3 is a view showing a configuration of a surgical tool of FIG. 1; FIG. 4 is a view showing a disposition example of coils incorporated in a coil unit of FIG 1; FIG. 5 is a configurational view showing a configuration of a luminal organ shape detecting apparatus of FIG. 1; FIG. 6 is a view showing configurations of a reception block and a control block of FIG. 5; FIG 7 is a view showing a detailed configuration of the reception block of FIG. 5; FIG. 8 is a timing view showing a working of a two-port memory and the like of FIG 6; FIG. 9 is a flowchart describing an action of the luminal organ shape detecting apparatus of FIG. 1; FIG. 10 is an explanatory view describing processings of FIG 9; FIG 11 is a view showing a configuration of a first modification example of a probe of FIG 1; and FIG. 12 is a view showing a configuration of a second modification example of the probe of FIG 1.

As shown in FIG. 1, a surgery system 1 as a surgery assisting apparatus according to the present example includes a surgery apparatus 2 for performing treatment on a region to be treated in a body of a patient 5 by abdominal operation procedures, and a luminal organ shape detecting apparatus 3 used for assisting (supporting) the abdominal operation procedures. The luminal organ shape detecting apparatus 3 is used as blood vessel position notifying means when performing the abdominal operation procedures by inserting a probe 15 as a luminal organ insertion probe in a blood vessel, for example, of a patient 5 lying on a bed 4.

The surgery apparatus 2 includes, for example, a high-frequency cauterizing apparatus 103 for supplying high-frequency current, and a surgical tool 100 as a treatment instrument for cauterizing a region to be treated in a body of the patient 5 with high-frequency current supplied from the high-frequency cauterizing apparatus 103. The high-frequency cauterizing apparatus 103 and the surgical tool 100 are connected by a cable 102.

As shown in FIG. 2, the probe 15 is configured of an elongated flexible guide wire, and includes inside the guide wire along from a distal end to a proximal end, for example, sixteen magnetic-field generating elements (or source coils) 14a, 14b, ..., 14p (hereinafter generically shown by the reference symbol 14i: note that the number of source coils is not limited to sixteen). Furthermore, as shown in FIG. 3, the surgical tool 100 includes a magnetic-field generating element (or a source coil) 140 in the vicinity of a distal end thereof to which an electrode 110 as a treatment portion is provided.

Returning to FIG. 1, a source cable 16 extended from a rear end of the probe 15 has at a rear end thereof a connector 16a detachably connected to a detecting apparatus (also referred to as an apparatus main body) 21 as detecting means which is an apparatus main body of the luminal organ shape detecting apparatus 3. Similarly, a source cable 101 extended from a rear end of the surgical tool 100 has a rear-end connector 101a detachably connected to the detecting apparatus 21 of the luminal organ shape detecting apparatus 3.

Then, a driving signal is applied to the source coils 14i and 140 serving as magnetic-field generating means via the source cables 16 and 101 as driving signal transmission means from the detecting apparatus 21 side, and thereby the source coils 14i and 140 generate magnetic fields.

In addition, the detecting apparatus 21 disposed near the bed 4 on which the patient 5 is lying has a (sense) coil unit 23 provided movably (ascendably and descendably) in up and down direction and a plurality of magnetic-field detecting elements (sense coils) in the coil unit 23.

More particularly, as shown in FIG. 4, twelve sense coils are arranged in such a manner that: sense coils 22a-1, 22a-2, 22a-3, and 22a-4 are oriented in the direction of, for example, an X axis and the Z coordinates of the centers of the coils are located on, for example, a first Z coordinate; sense coils 22b-1, 22b-2, 22b-3, and 22b-4 are oriented in the direction of a Y axis and the Z coordinates of the centers of the coils are located on a second Z coordinate different from the first Z coordinate; and sense coils 22c-1, 22c-2, 22c-3, and 22c-4 are oriented in the direction of a Z axis and the Z coordinates of the centers of the coils are located on a third Z coordinate different from the first and the second Z coordinates (hereinafter, the twelve sense coils are generically shown by the reference symbol 22j).

The sense coils 22j are connected to the detecting apparatus 21 via a cable 23a extended from the coil unit 23. The detecting apparatus 21 includes an operation panel 24 for a user to operate the apparatus. In addition, the detecting apparatus 21 has a liquid crystal monitor 25 provided at an upper part thereof as display means for displaying a detected luminal organ shape (hereinafter, referred to as a probe image) and a distal end position of the surgical tool 100 (hereinafter referred to as a tool distal end image).

As shown in FIG 5, the luminal organ shape detecting apparatus 3 includes a transmission block 26 for driving the source coils 14i and 140, a reception block 27 for receiving signals received by the sense coils 22j in the coil unit 23, and a control block 28 for processing signals detected in the reception block 27.

As shown in FIG. 6, the probe 15 includes sixteen source coils 14i for generating magnetic fields arranged at a predetermined interval, as described above, and these source coils 14i and the source coil 140 are connected to a source coil driving circuit 31 for generating driving signals of seventeen different frequencies which configures the transmission block 26.

The source coil driving circuit section 31 drives each of the source coils 14i in the probe 15 and the source coil 140 in the surgical tool 100 by sine-wave driving signals of different frequencies and the respective driving frequencies are set based on driving frequency setting data (also referred to as driving frequency data) stored in driving frequency setting data storing means or driving frequency setting data memorizing means, not shown, in the source coil driving circuit section 31. The driving frequency data is stored in the driving frequency data storing means (not shown) in the source coil driving circuit section 31 by a CPU (central processing unit) 32 serving as shape estimating means for performing calculation processing of the probe shape in the control block 28, via a PIO (parallel input-output circuit) 33.

On the other hand, the twelve sense coils 22j in the coil unit 23 are connected to a sense coil signal amplifying circuit section 34 configuring the reception block 27.

In the sense coil signal amplifying circuit section 34, as shown in FIG. 7, twelve single-core coils 22k configuring the sense coils 22j are respectively connected to amplifying circuits 35k, thereby providing a processing system with twelve systems. Minute signals detected by the respective single-core coils 22k are amplified by the amplifying circuits 35k. Filter circuits 36k have bands through which a plurality of frequencies generated by source coil groups pass and remove unnecessary components. Then, outputs of the filter circuits 36k are provided to output buffers 37k to be converted into digital signals readable by the control block 28 by ADCs (analog-digital converters).

Note that, the reception block 27 includes the sense coil signal amplifying circuit section 34 and the ADCs 38k and the sense coil signal amplifying circuit section 34 includes the amplifying circuits 35k, the filter circuits 36k, and the output buffers 37k.

Returning to FIG. 6, outputs of the twelve systems in the sense coil signal amplifying circuit section 34 are transmitted to the twelve ADCs 38k, to be converted into digital data sampled at a predetermined sampling cycle based on a clock supplied from the control signal generating circuit section 40 as numerical value data writing means in the control block 28. The digital data is written into a two-port memory 42 as data outputting means via a local data bus 41 in response to a control signal from the control signal generating circuit section 40.

Note that, as shown in FIG. 7, the two-port memory 42 is functionally composed of a local controller 42a, a first RAM 42b, a second RAM 42c, and a bus switch 42d, and at a timing shown in FIG 8, the ADCs 38k start A/D conversion in response to an A/D conversion start signal from the local controller 42a. Then, in response to switching signals from the local controller 42a, the bus switch 42d switches between the RAM 42b and 42c such that the first RAM 42b and 42c are alternately used as a read memory and write memory, and in response to read signal, the two-port memory 42 constantly takes data in after the power is applied.

Again, returning to FIG. 6, the CPU 32 reads out the digital data written into the two-port memory 42 in response to the control signal from the control signal generating circuit section 40 via an internal bus 46 composed of a local data bus 43, a PCI controller 44, and a PCI bus 45 (See FIG. 7). Then the CPU 32, by using a main memory 47, performs a frequency extraction processing (fast Fourier transform: FFT) on the digital data to separate and extract the data into magnetic field detection information of frequency components corresponding to driving frequencies of the respective source coils 14i and the source coil 140. Then, the CPU 32 calculates spatial position coordinates of the respective source coils 14i provided in the probe 15 and the source coil 140 in the surgical tool 100 from the respective digital data of the separated magnetic field detection information.

Furthermore, the CPU 32 estimates an insertion state of the probe 15 and a position of the distal end of the surgical tool 100 from the calculated position coordinate data, and generates display data forming the probe image and tool distal end image to output the data to a video RAM 48. A video signal generating circuit 49 reads out the data written into the video RAM 48 and converts into an analog video signal to output to the liquid crystal monitor 25. When the analog video signal is inputted, the liquid crystal monitor 25 displays the probe image and the tool distal end image on a display screen.

The CPU 32 calculates the magnetic field detection information corresponding to the respective source coils 14i and the source coil 140, that is, electromotive force (amplitude values of sine-wave signals) generated in the single-core coils 22k configuring the respective sense coils 22j and phase information thereof. Note that the phase information shows positive and negative polarities of the electromotive force.

Description will be made on an action of the present example configured as such.

When abdominal operation procedure for treating a region to be treated in a body of the patient 5 is started by inserting the probe 15 in a blood vessel of the patient 5 and using the surgical tool 100 (See FIG. 1), as shown in FIG. 9, the detecting apparatus 21 of the luminal organ shape detecting apparatus 3 detects the positions of the respective source coils 14i in the probe 15 in step S1. Subsequently, in step S2, the detecting apparatus 21 detects the position of the source coil 140 of the surgical tool 100.

Next, in step S3, the detecting apparatus 21 generates the probe image and the tool distal end image based on the detected position information, and in step S4, as shown in FIG. 10, displays the probe image 150 and the tool distal end image 151 on the monitor 25.

The processings are repeated until termination of the procedure is detected in step S5.

Thus, in the present example, the positional relation between the blood vessel into which the probe 15 is inserted and the distal end of the surgical tool 100 can be clearly displayed by the probe image 150 and the tool distal end image 151 on the monitor 25. Accordingly, even if an operator cannot easily see the blood vessel to which attention should be paid when treating the region to be treated, the operator can easily recognize the blood vessel by visually checking the positional relation between the probe image 150 and the tool distal end image 151, thereby appropriately assisting the procedure.

Note that, in the present example, a shape of a blood vessel is detected by disposing the plurality of source coils 14i in the probe 15 which is inserted into a blood vessel and the like. However, the present example is not limited to the same, and as shown in FIG. 11, the plurality of source coils 14i may be disposed in a side wall of a hollow catheter 160 to detect the shape of the blood vessel. In addition, as shown in FIG. 12, the plurality of source coils 14i may be disposed on outer circumference of the catheter 160 not in the side wall of the hollow catheter 160. That is, the luminal organ insertion probe may be the catheter 160 shown in FIG. 11 or FIG. 12.

Furthermore, though description was made taking the blood vessel as an example of the luminal organ in the present example, it is needless to say that the luminal organ whose shape is detected may be a urinary tract, a bile duct, an intestinal tract, or the like, depending on a kind of procedure.

In a case where the luminal organ is a bile duct, intestinal tract, or the like, the endoscope, of which shape is detectable, disclosed in Japanese Unexamined Patent Application Publication No. 2003-290129 may be the luminal organ insertion probe instead of the probe 15.

### (Second Example)

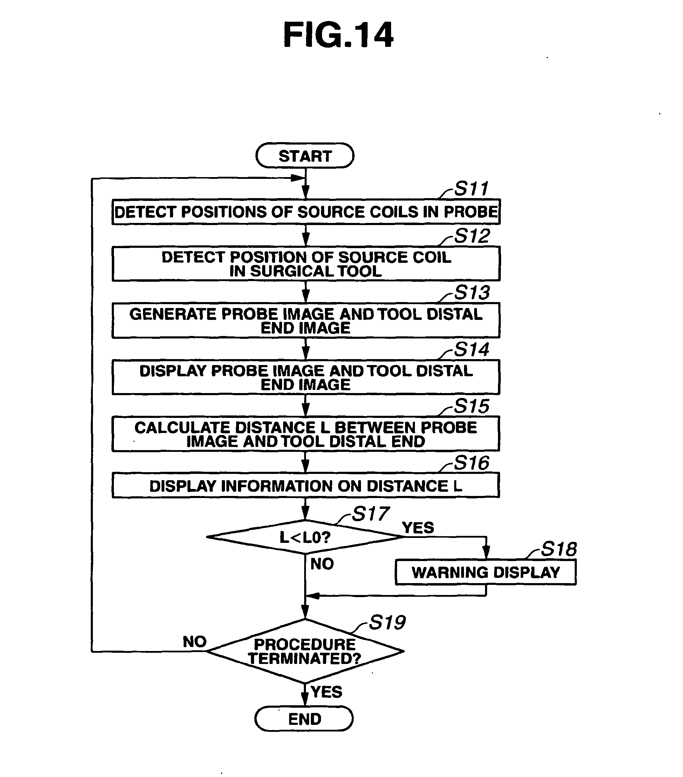

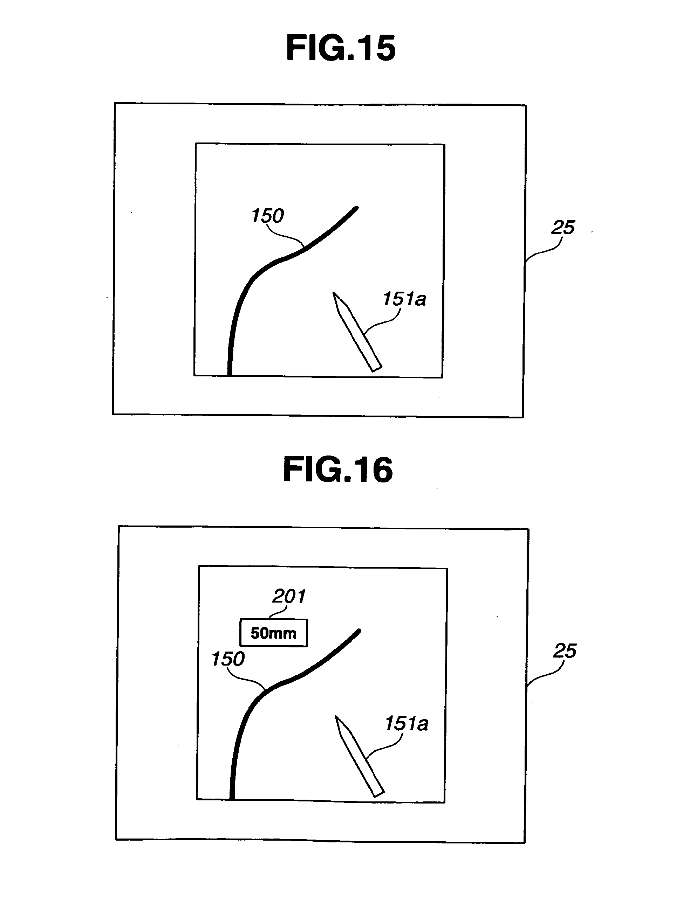

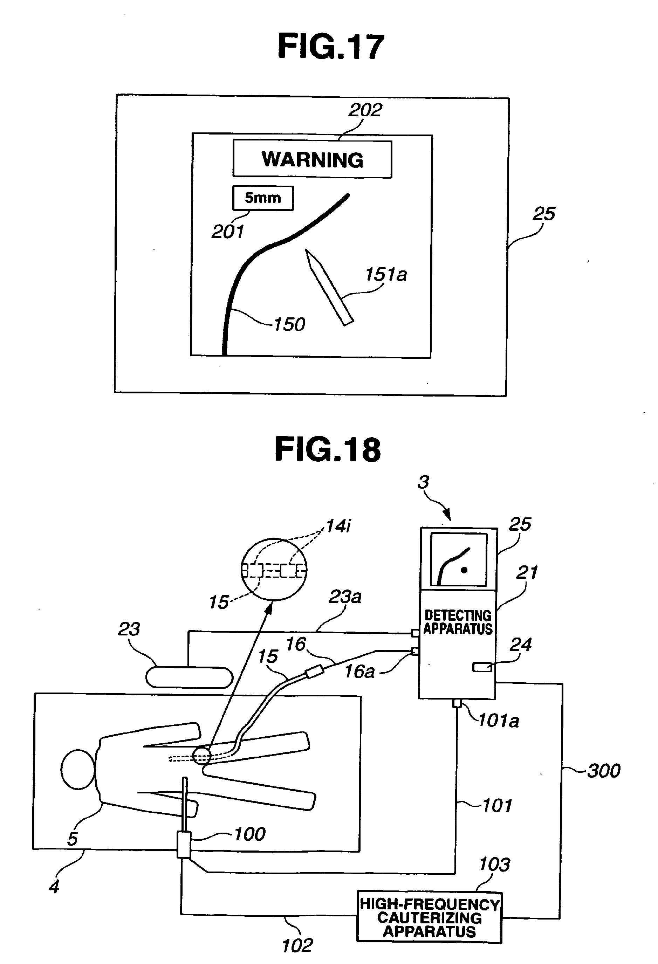

FIGS. 13 to 17 relate to the second example useful for understanding the present invention, in which: FIG. 13 is a view showing a configuration of a surgical tool; FIG. 14 is a flowchart describing an action of the luminal organ shape detecting apparatus when using the surgical tool of FIG. 13; FIG. 15 is a first explanatory view describing processings of FIG. 14; FIG. 16 is a second explanatory view describing the processings of FIG. 14; and FIG. 17 is a third explanatory view describing the processings of FIG.

The second example is almost the same as the first example, so that only the different points will be described. The same components are attached with the same reference symbols, and the descriptions thereof will be omitted.

As shown in FIG 13, the surgical tool 100 of the present example has, in the vicinity of the distal end thereof at which the electrode 110 is provided, a plurality of, or at least two source coils 140, 141 disposed along a longitudinal axis. By detecting the positions of the two source coils 140, 141, the position of the distal end of the surgical tool 100 and the orientation of the surgical tool 100 are detected. Other configurations are the same as those in the first example.

Description will be made on an action of the present example thus configured.

When abdominal operation procedure for treating a region to be treated in a body of the patient 5 is started by inserting the probe 15 in a blood vessel of the patient 5 and using the surgical tool 100 (See FIG. 1), as shown in FIG 14, the detecting apparatus 21 of the luminal organ shape detecting apparatus 3 detects the positions of the respective source coils 14i in the probe 15 in step S11. Subsequently, in step S12, the detecting apparatus 21 detects the positions of the source coils 140, 141 in the surgical tool 100.

Next, in step S 13, the detecting apparatus 21 generates the probe image and the tool distal end image based on the detected position information, to display the probe image 150 and the tool distal end image 151a on the monitor 25 in step S 14, as shown in FIG 15.

Note that, the orientation of the surgical tool 100 is calculated using the source coils 140, 141 in the present example. Accordingly, the position and the orientation of the surgical tool 100 can be known from the tool distal end image 151a, as shown in FIG 15.

Then, in step S15, the detecting apparatus 21 calculates the shortest distance L between the probe image and the tool distal end, to display distance information 201 indicating the distance L on the monitor 25 in step S16, as shown in FIG. 16.

Next, in step S 17, the detecting apparatus 21 judges whether or not the distance L is less than a predetermined distance L0. When the distance L is less than the predetermined distance L0, the detecting apparatus 21 executes warning display processing for displaying on the monitor 25 warning information 202 notifying that the blood vessel and the surgical tool 100 are in proximity to each other, in step S18, as shown in FIG. 17.

The above processings are repeated until termination of the procedure is detected in step S19.

Thus, in the present example, in addition to the effects of the first example, the orientation of the surgical tool 100 can be visually checked on the tool distal end image 151 a, so that the operator can recognize the proximity state between the blood vessel and the surgical tool 100.

In addition, the distance information 201 and the warning information 202 are displayed on the monitor 25, so that the operator can more surely recognize the proximity state.

Note that, the warning information 202 is displayed on the monitor 25, when the distance L is less than the predetermined distance L0. However, warning may be issued by a sound signal from a speaker and the like, not shown, or by emitting light from light-emitting means not shown (for example, a lamp or an LED provided to the detecting apparatus 21).

### (First Embodiment)

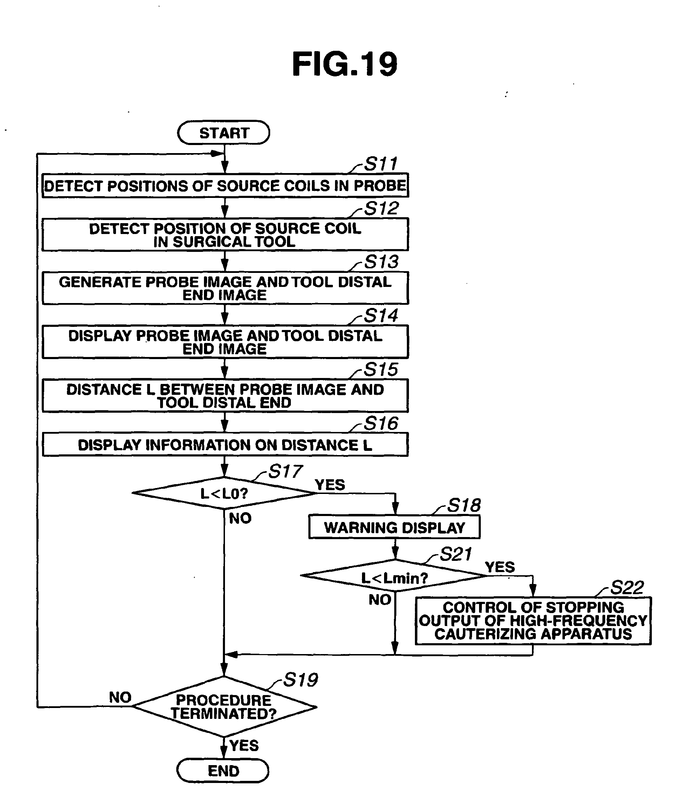

FIGS. 18 and 19 relate to the first embodiment of the present invention, in which FIG. 18 is a configurational view showing a configuration of a surgery system and FIG. 19 is a flowchart describing an action of a luminal organ shape detecting apparatus of FIG. 18.

The third embodiment is almost the same as the second example, so that only the different points will be described. The same components are attached with the same reference symbols, and the descriptions thereof will be omitted.

In the present embodiment, as shown in FIG 18, the detecting apparatus 21 of the luminal organ shape detecting apparatus 3 controls the output of the high-frequency cauterizing apparatus 103 via a control cable 300, depending on the proximity state between the blood vessel and the surgical tool 100. Other configurations are the same as those in the second example.

Description will be made on an action of the present embodiment thus configured.

As shown in FIG 19, step S11 to step S 18 are the same as those in the second example. In the present embodiment, after the warning display processing in step S 18, the detecting apparatus 21 judges in step S21 whether or not the distance L between the probe image and the tool distal end has become less than the limit minimum distance Lmin which is shorter than the predetermined distance L0. When judging the distance L is less than the limit minimum distance Lmin, the detecting apparatus 21 controls to stop the output of the high-frequency cauterizing apparatus 103 via the control cable 300 in step S22.

Other processings are the same as those in the second example, and the processings are repeated until the termination of procedure is detected in step S 19.

Thus, in the present embodiment, in addition to the effects of the second example, when the distance between the blood vessel and the distal end of the surgical tool 100 becomes less than the limit minimum distance Lmin which is shorter than the predetermined distance L0, the output of the high-frequency cauterizing apparatus 103 can be stopped.

### (Third Example)

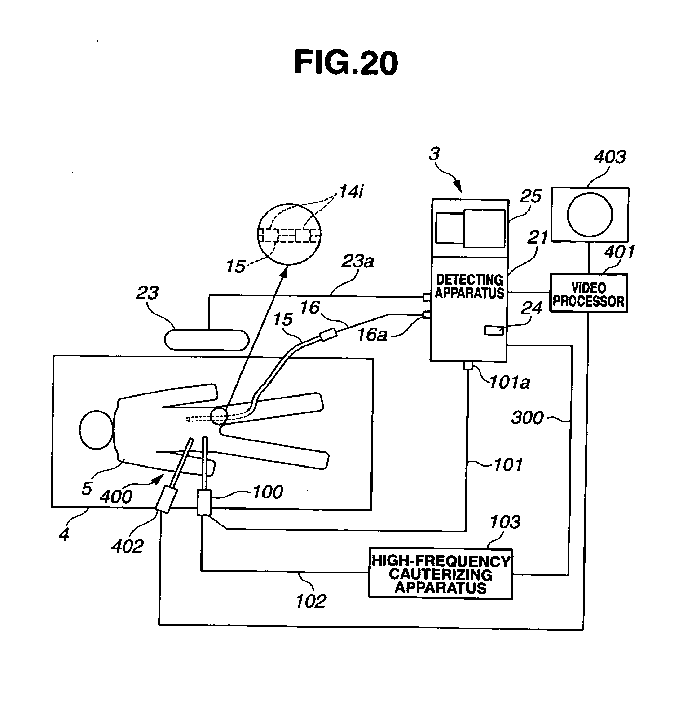

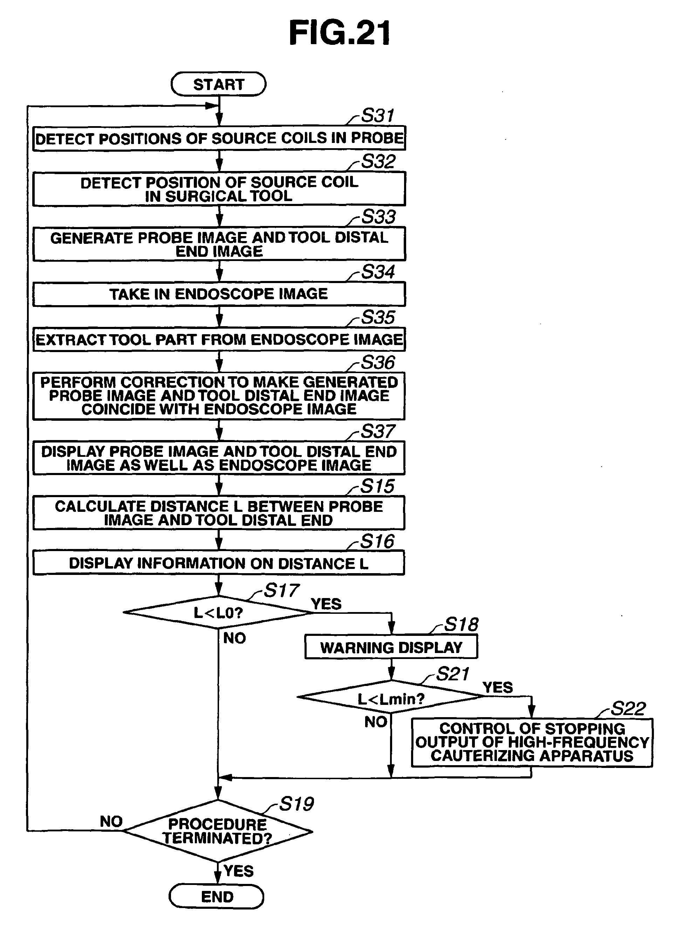

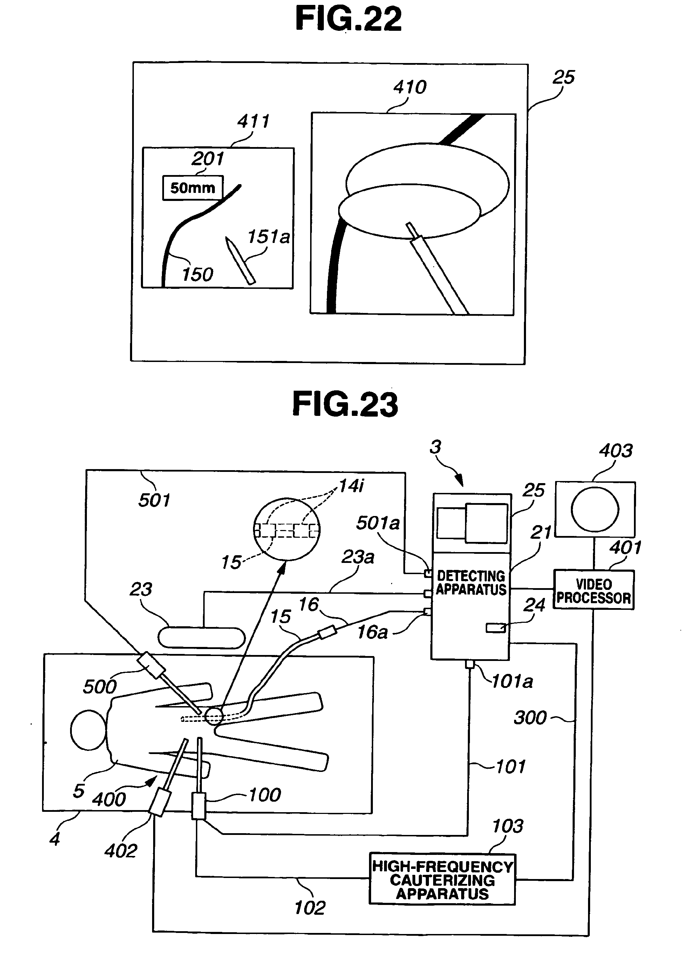

FIGS. 20 to 22 relate to the third example useful for understanding the present invention, in which: FIG. 20 is a configurational view showing a configuration of a surgery system; FIG. 21 is a flowchart describing an action of a luminal organ shape detecting apparatus of FIG 20; and FIG 22 is an explanatory view describing processings of FIG. 21.

The third example is almost the same as the first embodiment, so that only the different points will be described. The same components are attached with the same reference symbols, and the descriptions thereof will be omitted.

In the above examples and embodiment, description was made taking the abdominal operation as examples. However, in the present example, an example applied to a low invasive laparoscopic procedure will be described.

As shown in FIG 20, in the present example, there is provided a laparoscope 400 to be inserted in an abdominal cavity via a trocar not shown. Note that also the surgical tool 100 is inserted into an abdominal cavity via a trocar not shown.

The laparoscope 400 has a light guide (not shown) inserted therein, and the light guide transmits illumination light from a light source portion in a video processor 401 and emits the transmitted illumination light from an illumination window provided at the distal end of the insertion portion to illuminate a target region and the like of the patient 5. An image of an illuminated subject such as the target region is formed by an eyepiece portion via an objective lens, a relay lens, and the like, mounted to an observation window provided adjacent to the illumination window. At the image-forming position, a camera head 402 is detachably provided, and the image is formed on the image pickup device (CCD) which performs photoelectrical conversion.

The photoelectrically converted signal is signal-processed by a video signal processing section in the video processor 401, thereby a standard video signal being generated and displayed on a monitor 403 for image observation connected to the video processor 401. In addition, from the video processor 401, an endoscope image data of the subject such as the target region is outputted to the detecting apparatus 21 of the luminal organ shape detecting apparatus 3. Other configurations are the same as those in the first embodiment.

Description will be made on an action of the present example thus configured.

When treatment by the laparoscopic procedure is started by inserting the probe 15 into the blood vessel of the patient 5 and guiding the laparoscope 400 and the surgical tool 100 via the trocar into a region to be treated in the body of the patient 5, as shown in FIG. 21, in step S31, the detecting apparatus 21 of the luminal organ shape detecting apparatus 3 detects the positions of the respective source coils 14i in the probe 15. Subsequently, in step S32, the detecting apparatus 21 detects the positions of the source coils 140, 141 in the surgical tool 100.

Next, in step S33, the detecting apparatus 21 generates the probe image and the tool distal end image based on the detected position information.

Subsequently, the detecting apparatus 21 takes in the endoscope image data of the subject such as the target region picked up by the camera head 402 in step S34, and image-processes the taken-in endoscope image data to extract an image part of the surgical tool 100, for example, in step S35.

Then, in step S36, the detecting apparatus 21 corrects the orientations of the probe image and the tool distal end image such that the tool distal end image coincides with the image position of the extracted image part of the surgical tool 100.

Then, in step S37, the detecting apparatus 21 displays the taken-in endoscope image data in a live image display area 410 on the monitor 25, and also displays the probe image 150 and the tool distal end image 151 a in a shape display area 411 on the monitor 25, as shown in FIG. 22. At this time, due to the correction in step S36, the tool distal end image 151a displayed in the shape display area 411 and the surgical tool 100 displayed in the live image display area 410 are images located at relatively the same position and oriented in relatively the same direction in each area, and the dispositions of the probe image 150 and the tool distal end image 151a which are displayed in the shape display area 411 coincide with the endoscope image data displayed in the live image display area 410.

The subsequent processings after step S15 are the same as those in the first embodiment.

Thus, in the present example, similar effects as those in the first embodiment can be obtained also in the laparoscopic procedure.

Note that the present example may be applied not only to the laparoscope but also to an electronic endoscope including a flexible insertion portion, for example. In this case, the surgical tool is one inserted into a treatment instrument channel of the electronic endoscope, and it is needless to say that the same action and effects as those in the present example an be obtained by providing a source coil to a distal end of this tool

### (Fourth Example)

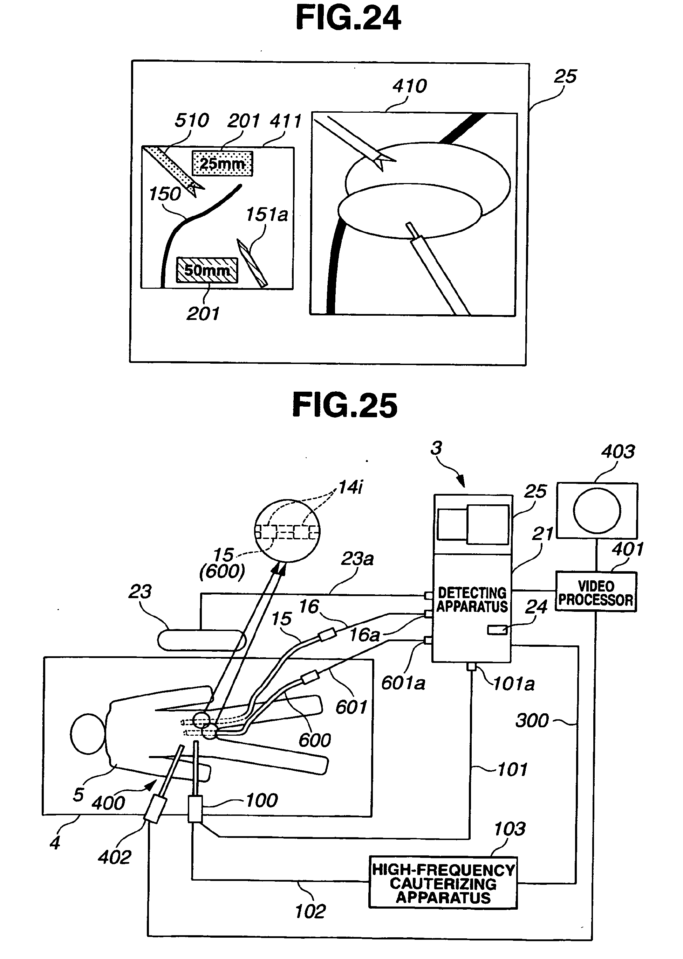

FIGS. 23 and 24 relate to the fourth example useful for understanding the present invention, in which FIG. 23 is a configurational view showing a configuration of a surgery system and FIG. 24 is an explanatory view describing an action of a luminal organ shape detecting apparatus of FIG 23.

The fourth example is almost the same as the third example, so that only the different points will be described. The same components are attached with the same reference symbols, and the descriptions thereof will be omitted.

As shown in FIG 23, the present example shows an example in which, in addition to the surgical tool 100, a second surgical tool 500 is inserted into an abdominal cavity, via a trocar, not shown.

The second surgical tool 500 is a grasping forceps and the like, for example, and is provided with the source coils 140, 141 in the vicinity of the distal end grasping portion similarly as the surgical tool 100, though not shown. The source coils 140, 141 are detachably connected to the detecting apparatus 21 of the luminal organ shape detecting apparatus 3 with a connector 501 a of the source cable 501 extended from a rear end of the surgical tool 500, to be driven similarly as the source coils 140, 141 in the surgical tool 100.

Other configurations are the same as those in the third example.

In the present example, the same processings (see FIG 21) as those in the third example are performed. However, as shown in FIG 24, in the shape display area 411 on the monitor 25, in addition to the probe image 150 and the tool distal end image 151 a of the surgical tool 100, the tool distal end image 510 of the surgical tool 500 is displayed. At this time, display shapes are generated for each tool so as to distinguish between the tool distal end image 151a and the tool distal end image 510.

In addition, in order to more clearly distinguish between the tool distal end image 151a and the tool distal end image 510, the images may be displayed in different colors, and the like. In this case, the distance information 201 is displayed matching with the color of the tool distal end image. Note that, in a case of also displaying the warning information 202 (see FIG 17), the information is displayed matching with the color of the tool distal end image as a warning object.

Thus, in the present example, in addition to the effects in the third example, it is possible to appropriately assist the procedures also when a plurality of surgical tools are employed.

### (Fifth Example)

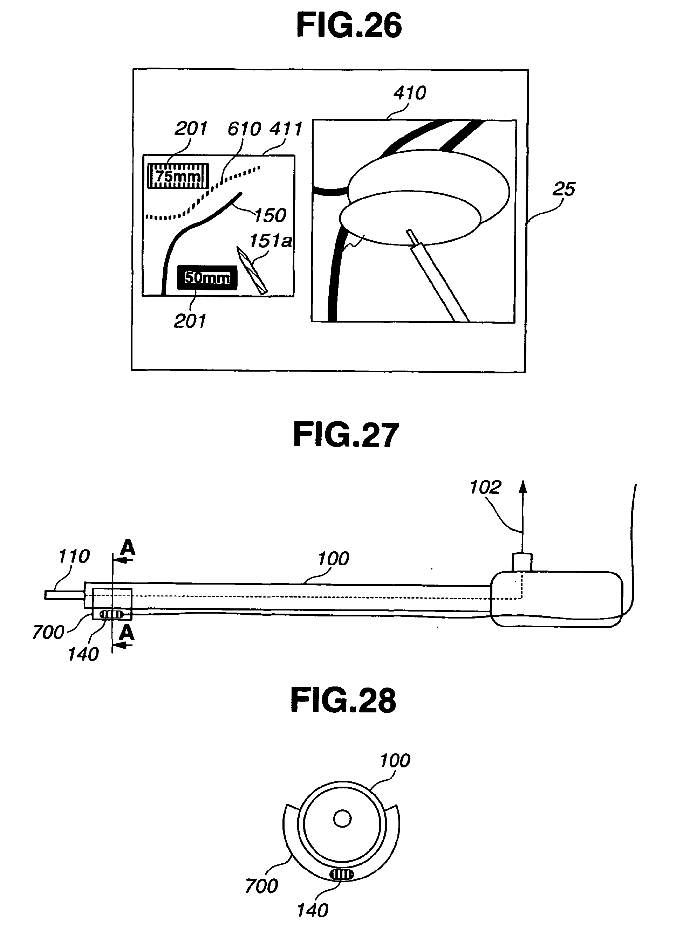

FIGS. 25 and 26 relate to the fifth example useful for understanding the present invention, in which FIG. 25 is a configurational view showing a configuration of a surgery system and FIG. 26 is an explanatory view describing an action of a luminal organ shape detecting apparatus of FIG. 25.

The fifth example is almost the same as the third example, so that only the different points will be described. The same components are attached with the same reference symbols, and the descriptions thereof will be omitted.

As shown in FIG. 25, the present example is an example in which, in addition to the probe 15, used is a second probe 600 for detecting a shape of a blood vessel to which attention should be paid other than the blood vessel whose shape is detected by the probe 15.

The second probe 600 is configured similarly as the probe 15, and source coils 14i in the second probe 600 are detachably connected to the detecting apparatus 21 of the luminal organ shape detecting apparatus 3 by a connector 601a of a source cable 601 extended from a rear end of the probe 600, to be driven similarly as the source coils 14i of the probe 15.

Other configurations are the same as those in the third example.

In the present example, the same processings (see FIG. 21) as those in the third example are performed. As shown in FIG 26, on the shape display area 411 on the monitor 25, a probe image 610 of the second probe 600 is displayed, in addition to the probe image 150 of the probe 15 and the tool distal end image 151a of the surgical tool 100. At this time, the probe image 150a and the probe image 610 are distinguishably displayed in different colors. In addition, in this case, the distance information 201 is displayed matching the color of the tool distal end image. Note that, also in a case of displaying the warning information 202 (see FIG 17), the warning information 202 is displayed matching the color of the tool distal end image.

Thus, in the present example, in addition to the effects of the third example, even in a case where there are a plurality of luminal organs such as blood vessel to which attention should be paid, it is possible to appropriately assist the procedures by disposing probes provided with the source coils 14i in a plurality of luminal organs and detecting the shapes thereof.

### (Sixth Example)

FIGS. 27 and 28 relate to the sixth example useful for understanding the present invention, in which FIG. 27 is a view showing a configuration of a surgical tool, and FIG. 28 is a cross-sectional view showing a cross section cut along A-A line of FIG. 27.

The sixth example is almost the same as the first example, so that only the different points will be described. The same components are attached with the same reference symbols, and the descriptions thereof will be omitted.

In the present example, as shown in FIGS. 27 and 28, at the distal end portion of the surgical tool 100, there is attachably provided a magnetic coil unit 700 having a source coil 140 incorporated in a mounting portion which takes advantage of a spring characteristic of material, for example.

Other configurations are the same as those in the first example, so that the present example can obtain the same action and effects of those in the first example.

Note that the way of mounting the magnetic coil unit 700 to the surgical tool 100 is not limited to the above, and other fixing means may be employed. Furthermore, the source coil 140 may be detachable from the magnetic coil unit 700.

In addition, a plurality of magnetic coil units 700 may be set in the surgical tool 100.

### (Seventh Example)

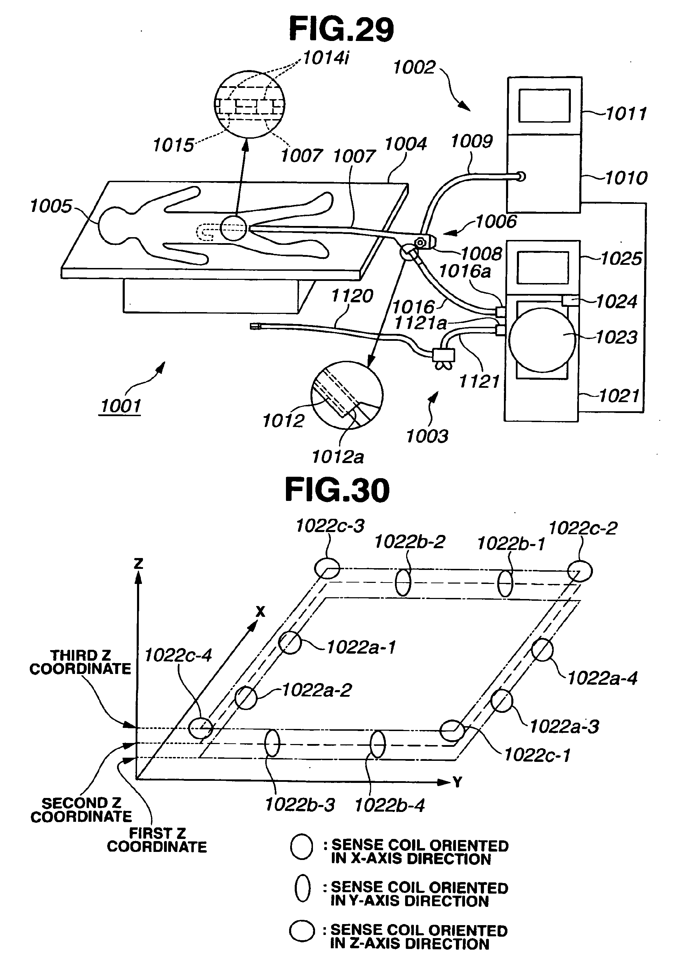

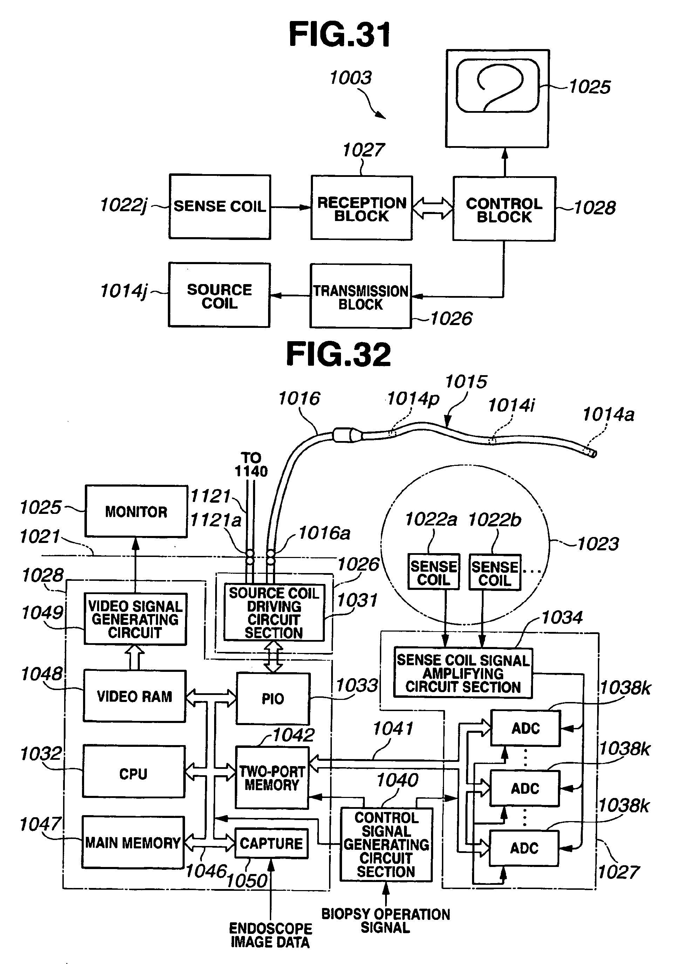

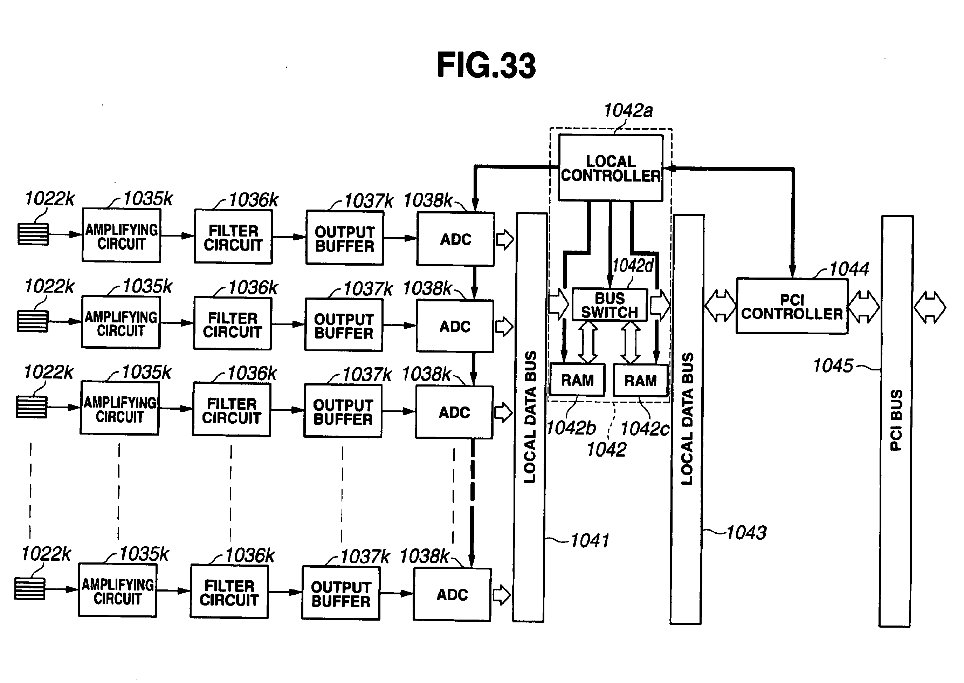

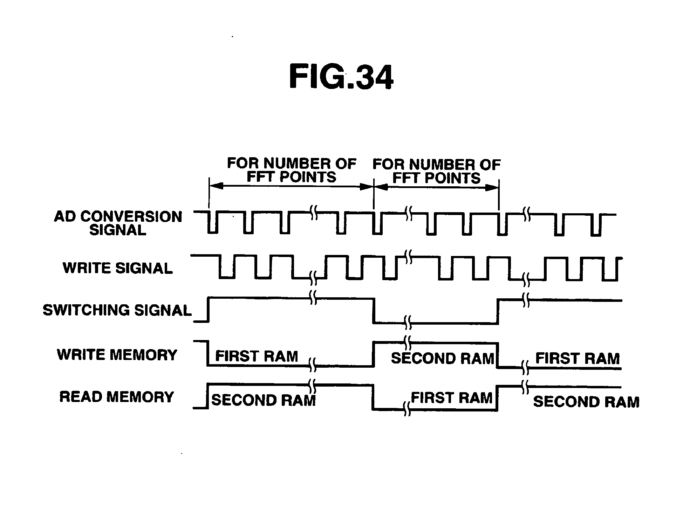

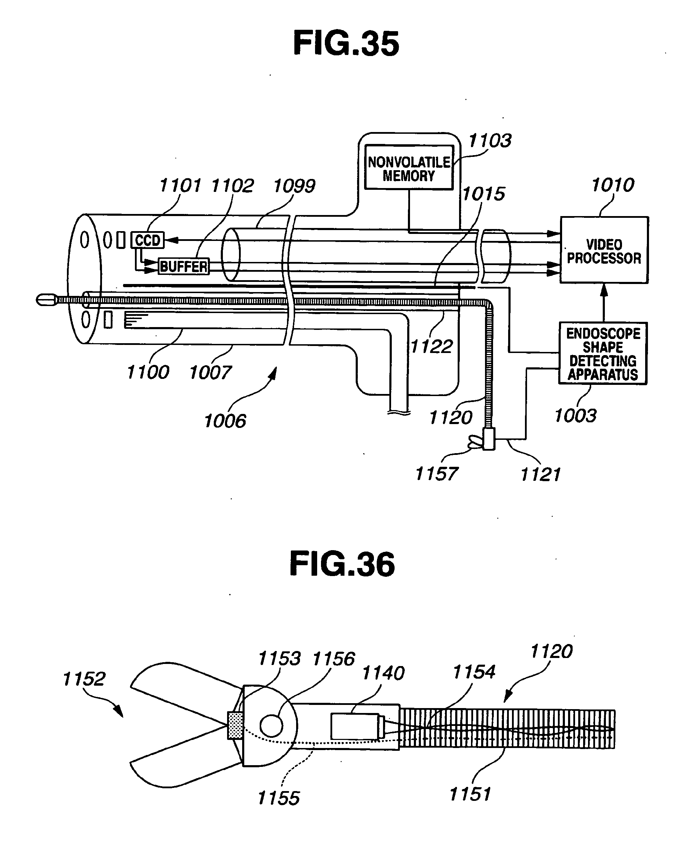

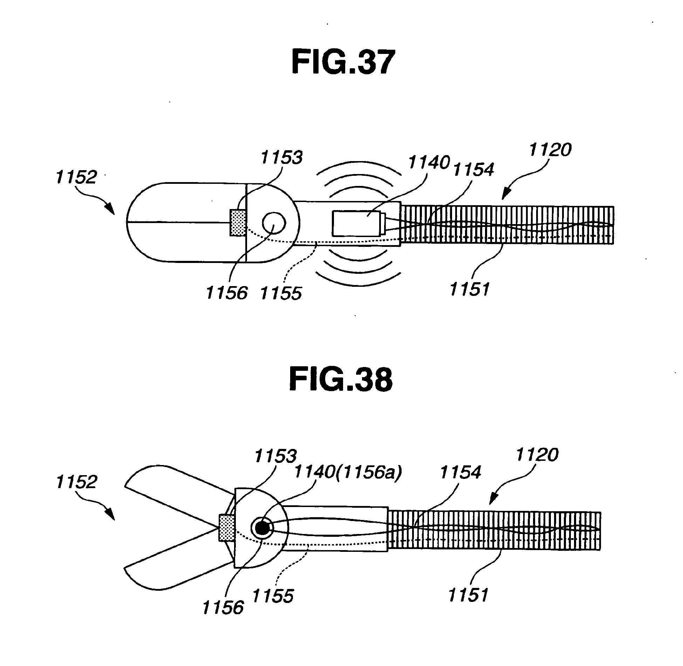

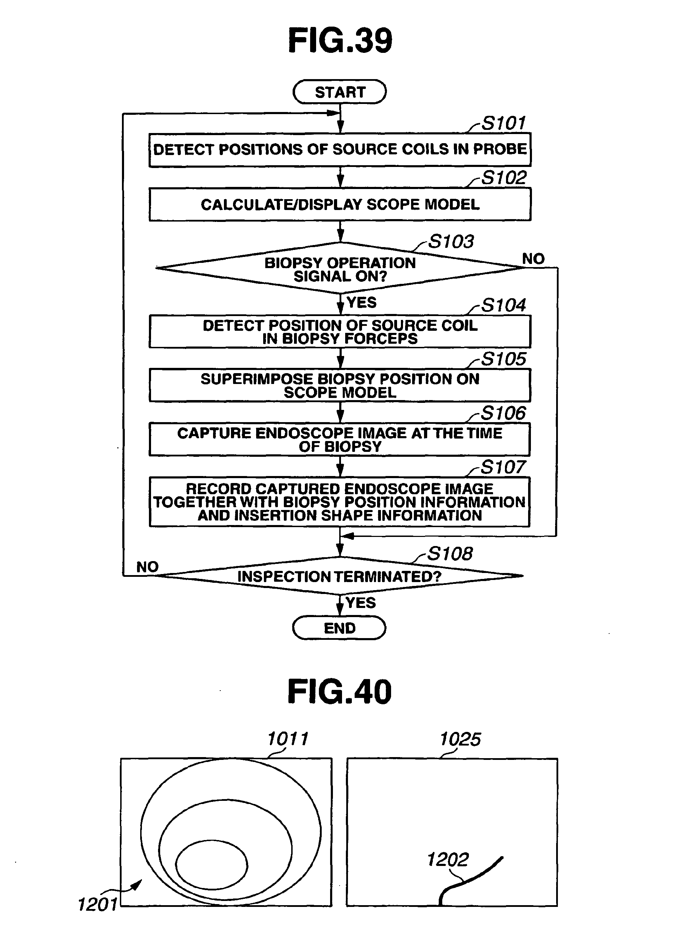

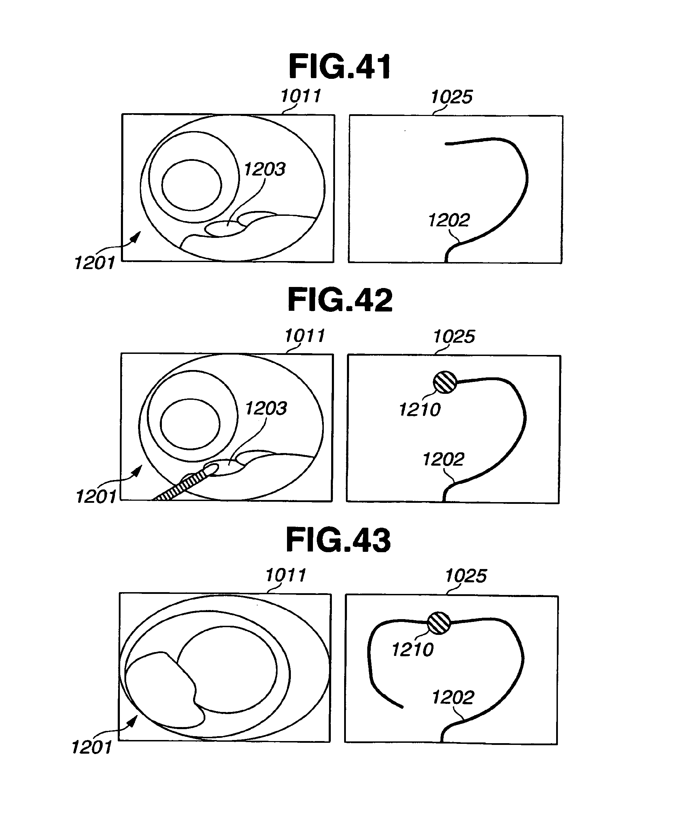

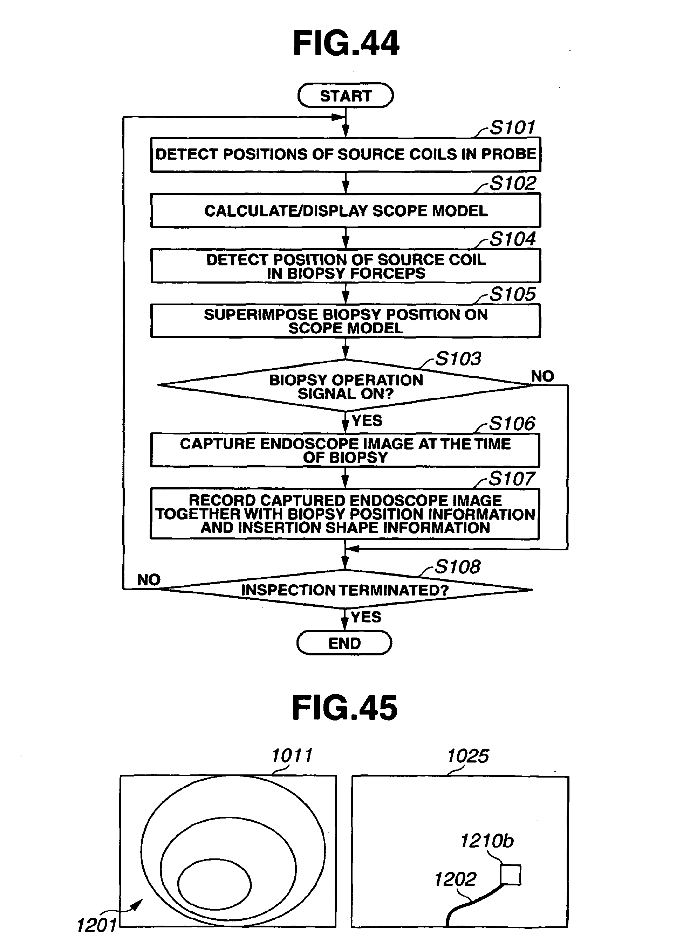

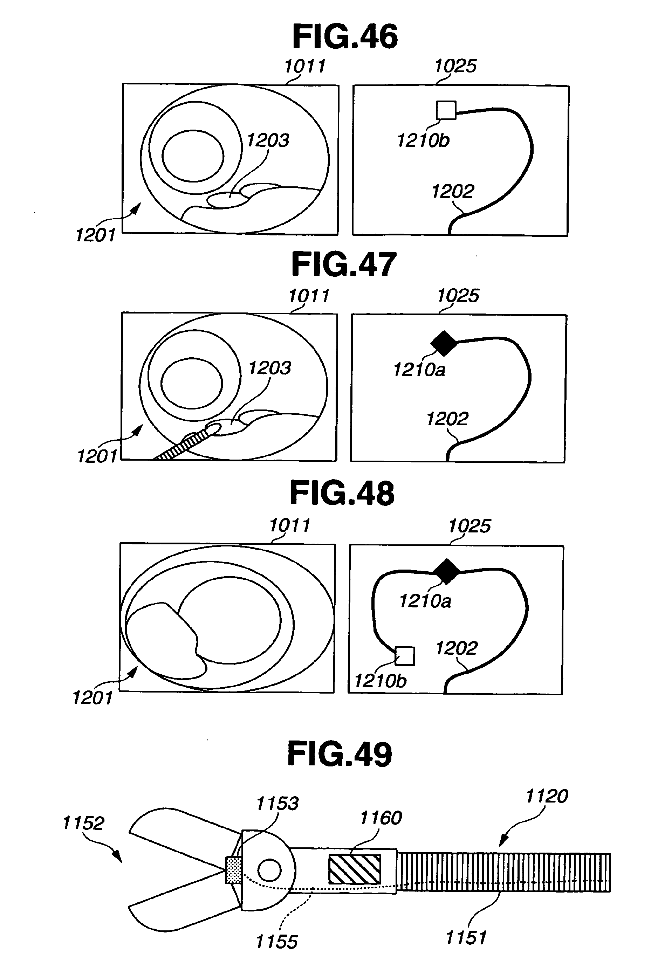

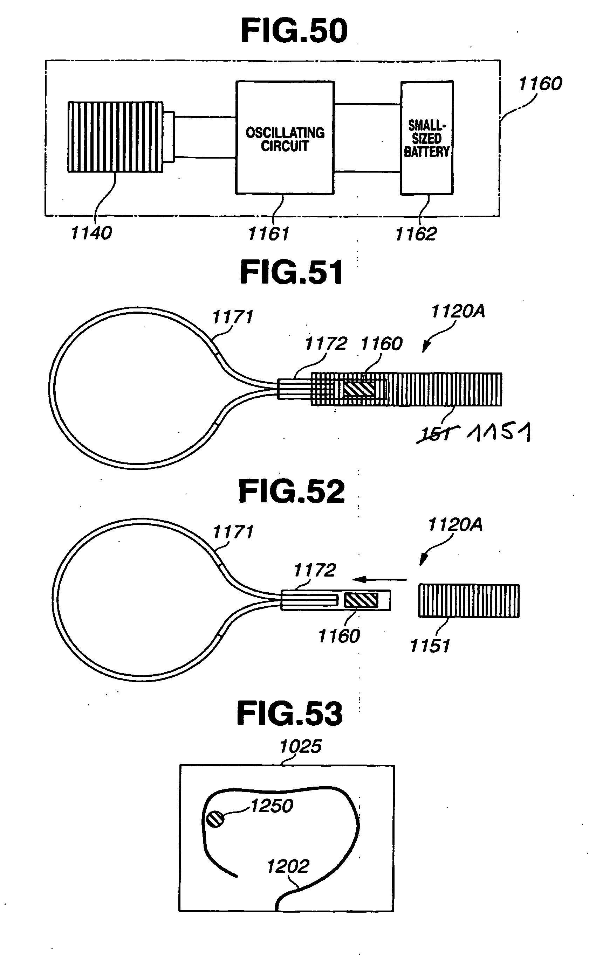

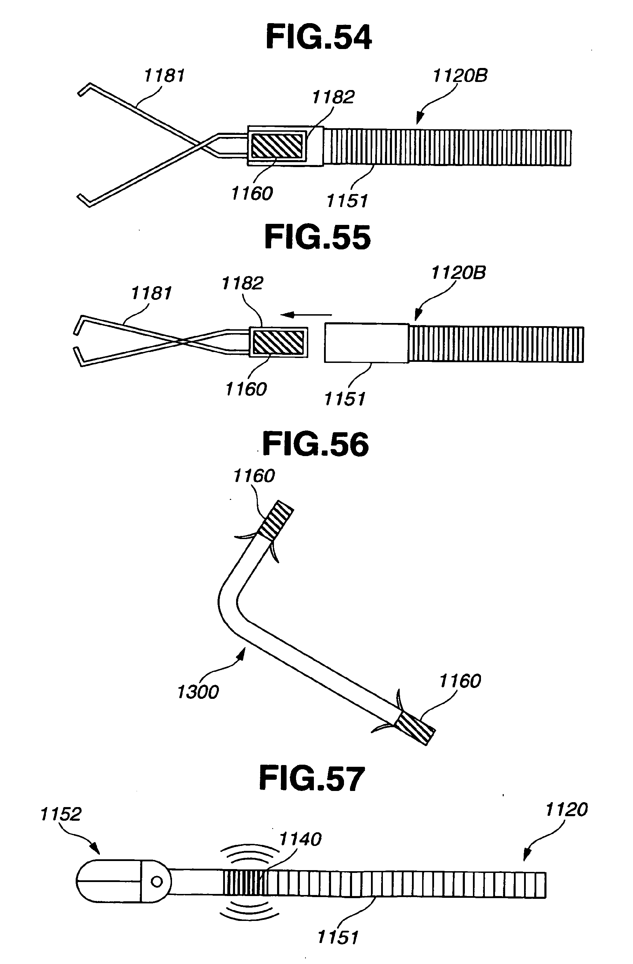

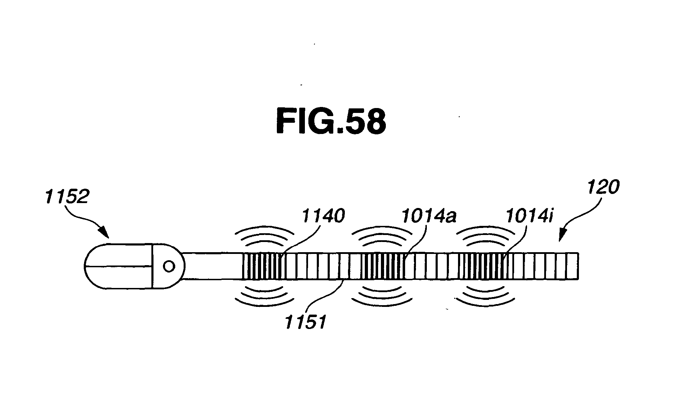

FIGS. 29 to 58 relate to the seventh example useful for understanding the present invention, in which: FIG. 29 is a configurational view showing a configuration of an endoscope system; FIG. 30 is a view showing a disposition example of coils incorporated in a coil unit of FIG. 29; FIG. 31 is a configurational view showing a configuration of an endoscope shape detecting apparatus of FIG. 29; FIG. 32 is a view showing configurations of a reception block and a control block of FIG. 31; FIG. 33 is a view showing a detailed configuration of the reception block of FIG. 31; FIG. 34 is a timing view showing a working of a two-port memory and the like of FIG. 32; FIG. 35 is a view showing a configuration of an electronic endoscope of FIG. 29; FIG. 36 is a first view showing a configuration of a biopsy forceps as a treatment instrument of FIG. 29; FIG. 37 is a second view showing a configuration of the biopsy forceps of FIG. 29; FIG. 38 is a view showing a configuration of a first modification example of the biopsy forceps of FIG. 37; FIG. 39 is a flowchart describing an action of the endoscope shape detecting apparatus of FIG. 29; FIG. 40 is a first view describing processings of FIG. 39; FIG. 41 is a second view describing the processings of FIG. 39; FIG. 42 is a third view describing the processings of FIG 39; FIG. 43 is a fourth view describing the processings of FIG. 39; FIG. 44 is a flowchart describing a modification example of an action of the endoscope shape detecting apparatus of FIG. 29; FIG. 45 is a first view describing processings of FIG. 44; FIG. 46 is a second view describing the processings of FIG. 44; FIG. 47 is a third view describing the processings of FIG. 44; FIG. 48 is a fourth view showing the processings of FIG. 44; FIG. 49 is a view showing a configuration of a second modification example of the biopsy forceps of FIG 37; FIG. 50 is a view showing a configuration of a source coil portion of FIG. 49; FIG. 51 is a first view showing a first modification example of a treatment instrument of FIG. 29; FIG. 52 is a second view showing the first modification example of the treatment instrument of FIG. 29; FIG 53 is a view describing an action of the treatment instrument of FIG. 51; FIG. 54 is a first view showing a second modification example of the treatment instrument of FIG. 29; FIG. 55 is a second view showing the second modification example of the treatment instrument of FIG 29; FIG. 56 is a view showing a third modification example of the treatment instrument of FIG. 29; FIG. 57 is a view showing a configuration of a third modification example of the biopsy forceps of FIG. 37; and FIG. 58 is a view showing a configuration of a fourth modification example of the biopsy forceps of FIG 37.

As shown in FIG 29, the endoscope system 1001 of the present example includes an endoscope apparatus 1002 for performing endoscopy, and an endoscope shape detecting apparatus 1003 used for assisting the endoscopy. The endoscope shape detecting apparatus 1003 is used as inspection assisting means when performing the endoscopy by inserting an insertion portion 1007 of an electronic endoscope 1006 into a body cavity of a patient 1005 lying on a bed 1004.

The electronic endoscope 1006 has, at a rear end of the flexible elongated insertion portion 1007, an operation portion 1008 provided with a bending operation knob, and a universal cord 1009 is extended from the operation portion 8 to be connected to a video imaging system (or video processor) 1010.

The electronic endoscope 1006 has a light guide inserted thereto, which transmits illumination light from a light source portion in the video processor 1010 and emits the transmitted illumination light from an illumination window provided at a distal end of the insertion portion 1007 to illuminate a diseased part and the like. The illuminated subject such as the diseased part and the like is image-formed by an objective lens mounted to the observation window provided adjacent to the illumination window on an image pickup device (CCD) disposed at the image-forming position which performs photoelectrical conversion.

The photoelectrically converted signal is signal-processed by a video signal processing section in the video processor 1010, thereby a standard video signal being generated and displayed on a monitor for image observation 1011 connected to the video processor 1010.

The electronic endoscope 1006 is provided with two forceps channels 1012, 1122 (not shown: see FIG. 35). By inserting the probe 1015 including, for example, sixteen magnetic-field generating elements (or source coils) 1014a, 1014b, ..., 1014p (hereinafter, generically shown by the reference symbol 1014i) from an insertion port 1012a of the forceps channel 1012, the source coils 1014i are disposed in the insertion portion 1007.

A source cable 1016 extended from a rear end of the probe 1015 has at the rear end thereof a connector 1016a detachably connected to a detecting apparatus 1021 (also referred to as apparatus main body), which is detecting means, as an apparatus main body of the endoscope shape detecting apparatus 1003. Then, high-frequency signals (driving signals) are applied to the source coils 1014i serving as magnetic-field generating means via the source cable 1016 as high-frequency signal transmitting means from a side of the detecting apparatus 1021, and thereby the source coils 1014i radiate electromagnetic waves having electromagnetic fields therearound.

In addition, to the forceps channel 1122 (not shown: see FIG. 35) of the electronic endoscope 1006, a biopsy forceps 1120, which is a treatment instrument having a source coil 1140 (not shown: see FIG 36) at a distal end thereof, is insertable. A source cable 1121 extended from a rear end of the biopsy forceps 1120 has at a rear end thereof a connector 1121 a detachably connected to the detecting apparatus 1021 as the apparatus main body of the endoscope shape detecting apparatus 1003. Then, a high-frequency signal (driving signal) is applied to the source coil 1140 serving as magnetic-field generating means via the source cable 1121 as high-frequency signal transmitting means from the side of the detecting apparatus 1021, and thereby the source coil 1140 radiates electromagnetic wave having an electromagnetic field therearound. Note that a detailed configuration of the biopsy forceps 1120 will be described later.

In addition, the detecting apparatus 1021 disposed near the bed 1004 on which the patient 1005 lies down has a (sense) coil unit 1023 provided movably (ascendably and descendably) in up and down direction and a plurality of magnetic-field detecting elements (sense coils) in the coil unit 1023.

More particularly, as shown in FIG. 30, twelve sense coils are arranged in such a manner that: sense coils 1022a-1, 1022a-2, 1022a-3, and 1022a-4 are oriented in the direction of, for example, an X axis and the Z coordinates of the centers of the coils are located on, for example, a first Z coordinate; sense coils 1022b-1, 1022b-2, 1022b-3, and 1022b-4 are oriented in the direction of a Y axis and the Z coordinates of the centers of the coils are located on a second Z coordinate different from the first Z coordinate; and sense coils 1022c-1, 1022c-2, 1022c-3, and 1022c-4 are oriented in the direction of a Z axis and the Z coordinates of the centers of the coils are located on a third Z coordinate different from the first and the second Z coordinates (hereinafter, the twelve sense coils are generically shown by the reference symbol 1022j).

The sense coils 1022j are connected to the detecting apparatus 1021 via a cable not shown extended from the coil unit 1023. The detecting apparatus 1021 has an operation panel 1024 for a user to operate the apparatus. Furthermore, the detecting apparatus 1021 has a liquid crystal monitor 1025 provided at an upper part thereof as display means for displaying a detected shape of the endoscope insertion portion (hereinafter referred to as a scope model).

As shown in FIG. 31, the endoscope shape detecting apparatus 1003, includes a transmission block 1026 for driving source coils 1014i, 1140, a reception block 1027 for receiving the signals received by the sense coils 1022j in the coil unit 1023, and a control block 1028 for signal processing the signal detected in the reception block 1027.

As shown in FIG. 32, the probe 1015 disposed in the insertion portion 1007 of the electronic endoscope 1006 includes sixteen source coils 1014i for generating magnetic fields provided at a predetermined interval, as described above, and these source coils 1014i are connected to a source coil driving circuit 1031, which configures the transmission block 1026 and generates driving signals of sixteen frequencies different from each other.

The source coil 1140 of the biopsy forceps 1120 is similarly connected to the source coil driving circuit 1031 to be driven by a driving signal of a frequency different from the frequencies of the driving signals for driving the source coils 1014i.

The source coil driving circuit 1031 drives each of the source coils 1014i and 1140 by sine-wave driving signals of different frequencies, respectively, the respective driving frequencies are set based on driving frequency setting data (also referred to as driving frequency data) stored in driving frequency setting data storing means or driving frequency setting data memorizing means, not shown, in the source coil driving circuit section 1031. The driving frequency data is stored in the driving frequency data storing means (not shown) in the source coil driving circuit section 1031 by a CPU (central processing unit) 1032 serving as shape estimating means for performing calculation processing of the endoscope shape and the like in the control block 1028, via a PIO (parallel input-output circuit) 1033.

On the other hand, the twelve sense coils 1022j in the coil unit 1023 are connected to a sense coil signal amplifying circuit section 1034 configuring the reception block 1027.

In the sense coil signal amplifying circuit section 1034, as shown in FIG. 33, twelve single-core coils 1022k configuring the sense coils 1022j are respectively connected to amplifying circuits 1035k, thereby providing a processing system with twelve systems. Minute signals detected by the respective single-core coils 1022k are amplified by the amplifying circuits 1035k. Filter circuits 1036k have bands through which a plurality of frequencies generated by source coil groups pass and remove unnecessary components. Then, outputs of the filter circuits 1036k are provided to output buffers 1037k to be converted by ADCs (analog-digital converters) 1038k into digital signals readable by the control block 1028.

Note that, the reception block 1027 includes the sense coil signal amplifying circuit section 1034 and the ADCs 1038k and the sense coil signal amplifying circuit 1034 includes the amplifying circuits 1035k, the filter circuits 1036k, and the output buffers 1037k.

Returning to FIG. 32, outputs of the twelve systems in the sense coil signal amplifying circuit section 1034 are transmitted to the twelve ADCs 1038k, to be converted into a digital data sampled at a predetermined sampling cycle based on a clock supplied from the control signal generating circuit section 1040 as numerical value data writing means in the control block 1028. The digital data is written into a two-port memory 1042 serving as data outputting means via a local data bus 1041 in response to a control signal from the control signal generating circuit section 1040.

Note that, as shown in FIG. 33, the two-port memory 1042 is functionally composed of a local controller 1042a, a first RAM 1042b, a second RAM 1042c, and a bus switch 1042d, and at a timing shown in FIG. 34, the ADCs 1038k start A/D conversion in response to an A/D conversion start signal from the local controller 1042a. Then, in response to a switching signal from the local controller 1042a, the bus switch 1042d switches between the RAM 1042b and 1042c such that the RAMS 1042b and 1042c are alternately used as a read memory and write memory, and in response to read signal, the two-port memory 1042 constantly takes data in after the power is applied.

Returning to FIG. 32 again, the CPU 1032 reads out the digital data written into the two-port memory 1042 in response to the control signal from the control signal generating circuit section 1040 via an internal bus 1046 composed of a local data bus 1043, a PCI controller 1044, and a PCI bus 1045 (See FIG. 33). Then the CPU 1032 performs a frequency extraction processing (fast Fourier transform: FFT) on the digital data by using a main memory 1047 to separate and extract the data into magnetic field detection information of frequency components corresponding to driving frequencies of the respective source coils 1014i and the source coil 1140. Furthermore, the CPU 1032 calculates spatial position coordinates of the respective source coils 1014i provided in the insertion portion 1007 of the electronic endoscope 1006 and the source coil 1140 of the biopsy forceps 1120 based on the respective digital data of the separated magnetic field detection information.

Also, the CPU 1032 estimates an insertion state of the insertion portion 1007 of the electronic endoscope 1006 from the calculated position coordinates data, and generates display data forming a scope model to output the display data to a video RAM 1048. A video signal generating circuit 1049 reads out the data written in the video RAM 1048 to convert into an analog video signal and outputs the video signal on the liquid crystal monitor 1025. When the analog video signal is inputted, the liquid crystal monitor 1025 displays the scope model of the insertion portion 1007 of the electronic endoscope 1006 on a display screen.

In addition, at the time of biopsy, the CPU 1032 estimates a biopsy position from the position coordinates data of the source coil 1140 in the biopsy forceps 1120 based on a biopsy operation signal, to display the biopsy position image on the scope model in a superimposed manner.

The CPU 1032 calculates magnetic field detection information corresponding to the respective source coils 1014i, 1140, that is, electromotive force (amplitude values of sine-wave signals) generated in the single-core coils 1022k configuring the respective sense coils 1022j and phase information thereof. Note that the phase information shows positive and negative polarities of the electromotive force.

When detecting an on-state (to be described later in detail) of the biopsy operation signal from the biopsy forceps 1120 via the control signal generating circuit section 1040, the CPU 1032 captures an endoscope image at that time from the video processor 1010 by a capture circuit 1050, triggered by the on-state of the biopsy operation signal, and records the captured endoscope image (still image) in the two-port memory 1042 together with the position coordinates data of the source coils 1014i and 1140.

As shown in FIG. 35, the electronic endoscope 1006 has in the insertion portion 1007 a light guide 1100 for transmitting illumination light and the probe 1015 having a plurality of source coils 1014i, and includes in the distal end portion of the insertion portion 1007 a CCD 1101 for picking up an image of a subject. Then, the CCD 1101 is driven in response to a driving signal from the video processor 1010, and the image pickup signal captured by the CCD 1101 is transmitted to the video processor 1010 via a buffer circuit 1102. The driving signal and the image pickup signal are transmitted and received between the video processor 1010 and the CCD 1101 via a signal cable 1099 inserted in the insertion portion 1007.

On the other hand, the electronic endoscope 1006 has, in an operation portion 1102 on a proximal end side thereof, a nonvolatile memory 1103 in which scope ID data and the like for identifying the electronic endoscope 1006 are stored. The nonvolatile memory 1103 is configured of the flash memory (registered trademark) and the like which are electrically rewritable. In addition, the electronic endoscope 1006 is provided with the forceps channel 1012 in which the probe 1015 is disposed, and the forceps channel 1122 in which the biopsy forceps 1120 is insertable.

As shown in FIG. 36, the biopsy forceps 1120 includes biopsy cups 1152 at a distal end of an elongated flexible coil shaft 1151. The biopsy cups 1152 are configured to be openable/closable with a hinge portion 1156 as a center by operating an operation portion 1157 (see FIG 35) provided at a proximal end of the biopsy forceps 1120. In the vicinity of the open/close center of the hinge portion 1156, an open/close sensor 1153 is provided, and the open/close sensor 1153 allows an open/close state of the biopsy cups 1152 to be detected. Also, the source coil 1140 is provided at a proximal end of the biopsy cups 1152. A detection signal from the open/close sensor 1153 and a driving signal of the source coil 1140 are transmitted to the endoscope shape detecting apparatus 1003 by a signal line 1155 and a signal line 1154, respectively, via the source cable 1121.

As shown in FIG 37, when the biopsy cups 1152 are closed and the detection signal from the open/close sensor 1153 becomes on-state (where, for example, the biopsy cups has changed from the close state to the open state), the endoscope shape detecting apparatus 1003 detects the detection signal as the biopsy operation signal. When detecting the on-state of the biopsy operation signal, the endoscope shape detecting apparatus 1003 drives the source coil 1140 to estimate the biopsy position from the position coordinates data of the source coil 1140.

Note that, as shown in FIG. 38, a hinge coil 1156a of the hinge portion 1156 can be used as the source coil 1140

An action of the present example thus configured will be described. When an inspection by the electronic endoscope 1006 is started, as shown in FIG. 39, the endoscope shape detecting apparatus 1003 drives the source coils 1014i in the probe 1015 disposed in the electronic endoscope 1006 to detect positions of the source coils 1014i (insertion shape information) by the sense coils 1022j in step S101, and estimates the insertion state of the insertion portion 1007 of the electronic endoscope 1006 to display a scope model on the liquid crystal monitor 1025 in step S102.

As a result, as shown in FIG. 40, an endoscope image 1201 picked up by the electronic endoscope 1006 is displayed on the monitor for image observation 1011, and a scope model 1202 showing the insertion state of the insertion portion 1007 of the electronic endoscope 1006 is displayed on the liquid crystal monitor 1025.

Then, the endoscope shape detecting apparatus 1003 judges whether or not the biopsy operation signal from the open/close sensor 1153 of the biopsy forceps 1120 is in the on-state in step S103. When the biopsy operation signal is in the on-state, processing proceeds to step S 104.

When the biopsy operation signal is in the off-state, processing proceeds to step S108, and the processings from step S101 to S 108 are repeated until the inspection is terminated in step S108.

Here, description will be made taking as an example a case where the electronic endoscope 1006 is continuously inserted in the body cavity and the display state changes from the display state of FIG. 40 to that of FIG. 41, and a living tissue 1203 of the endoscope image 1201 displayed on the monitor for image observation 1011 is biopsied.

As shown on the monitor for image observation 1011 in FIG. 41, when an operator biopsies the living tissue 1203 with the biopsy forceps 1120 while observing the monitor for image observation 1011, the biopsy operation signal becomes on-state in step S103. Then, in step S 104, the endoscope shape detecting apparatus 1003 drives the source coil 1140 disposed at the distal end of the biopsy forceps 1120, and detects a position of the source coil 1140 (biopsy position information) by the sense coils 1022j.

Then, in step S 105, as shown in FIG. 42, the endoscope shape detecting apparatus 1003 displays a biopsy position marker indicating the position of the source coil 1140 in a superimposed manner on the scope model 1202, as shown on the liquid crystal monitor 1025. Furthermore, in step S106, the endoscope shape detecting apparatus 1003 captures the endoscope image at this time by the capture circuit 1050.

Then, in step S107, the endoscope shape detecting apparatus 1003 records the captured endoscope image (still image) in the two-port memory 1042 together with the position of the source coil 1140 (biopsy position information) and the positions of the source coils 1014i (insertion shape information), and proceeds to step S108.

The processings described above are performed over a desired inspection area in the body cavity as shown in FIG 43, and continued until the inspection is terminated in step S108.

Note that, as shown in FIG 43, once a biopsy has been performed, the biopsy position marker 1210 is continued to be superimposed on the liquid crystal monitor 1025.

Thus, with the present example, the source coil 1140 is provided to the biopsy forceps 1120 as a treatment instrument, and the biopsy position is recorded triggered by the on-state of the biopsy operation signal, so that the position where a biopsy has been performed in the desired inspection area in the body cavity can be automatically recorded. In addition, the endoscope image at the time of the biopsy is captured to be recorded, therefore, the implementation state of the biopsy can be easily confirmed after the treatment.

Note that, though it was described that the captured endoscope image (still image) is recorded in the two-port memory 1042 together with the position of the source coil 1140 (biopsy position information) and the positions of the source coils 1014i (insertion shape information) in step S107, the present example is not limited to the same. At least only the position of the source coil 1140 (biopsy position information) and the positions of the source coils 1014i (insertion shape information) may be recorded.

In addition, in the present example, the source coil 1140 is driven, triggered by the on-state of the biopsy operation signal. However, the present example is not limited to the same. The source coil 1140 may be constantly driven in conjunction with the respective source coils 1014i of the probe 1015 to detect the position of the source coil 1140, and the biopsy position marker 1210 may be displayed in a superimposed manner on the liquid crystal monitor 1025. In this case, the processings in the endoscope shape detecting apparatus 1003 are as shown in FIG. 44, and the on-state of the biopsy operation signal, that is, a display form of the biopsy position marker at operation 1210a when the biopsy has been performed, and a display form of the biopsy position marker at a normal time 1210b when the operation is not performed can be changed as shown in FIGS. 45 to 48. This allows the position where the biopsy has been performed to be visually recognized easily. The biopsy position marker at operation 1210a is continuously displayed in a superimposed manner.

FIGS. 45 to 48 show an example in which the biopsy position marker at operation 1210a and the biopsy position marker at a normal time 1210b can be visually recognized by the display forms of ◆ and □, respectively. However, the display form may be changed by colors of the markers instead of the shapes of the markers, such that the biopsy position marker at operation 1210a is shown in red and the biopsy position marker at a normal time 1210b is shown in green. Or, the biopsy position marker at operation 1210a may be displayed in a constantly-lighted manner and the biopsy position marker at a normal time 1210b may be displayed in a blinking manner. Furthermore, the biopsy position marker at operation 1210a may be displayed such that the open/close state of the biopsy cups 1152 is displayed in animation.

In addition, in a case where the source coil 1140 is constantly driven in conjunction with the respective source coils 1014i of the probe 1015, as shown in FIG 49, a source coil portion 1160 which does not need a driving signal from outside may be provided instead of the source coil 1140. The source coil portion 1160 may include, as shown in FIG. 50, the source coil 1140, an oscillating circuit 1161 for driving the source coil, and a small-sized battery 1162.

The source coil portion 1160 can be applied not only to the above-described biopsy forceps 1120 but also to a detainment snare treatment instrument 1120A as shown in FIG. 51, for example. That is, in the detainment snare treatment instrument 1120A including a detainment snare portion 1171 connected to a distal end of a coil sheath 1151 via a connection member 1172, the source coil portion 1160 is disposed in the connection member 1172.

Then, as shown in FIG. 52, the connection member 1172 is detached from the coil sheath 1151 and the detainment snare portion 1171 is detained in a living tissue, not shown.

The position of the source coil portion 1160 is detected by the endoscope shape detecting apparatus 1003 in this state, thereby allowing the scope model 1202 and a detainment position image 1250 to be displayed on the liquid crystal monitor 1025, as shown in FIG. 53.

Similarly, the source coil portion 1160 can be applied also to a clip treatment instrument 1120B as shown in FIG 54. That is, in a clip treatment instrument 1120B including a clip portion 1181 connected to the distal end of a coil sheath 1151 via a connection member 1182, the source coil portion 1160 is disposed in the connection member 1182.

Then, as shown in FIG. 55, the connection member 1182 is separated from the coil sheath 1151 to clip a living tissue not shown with the clip portion 1181.

The position of the source coil portion 1160 is detected by the endoscope shape detecting apparatus 1003 in this state, thereby allowing the scope model 1202 and a clip position image to be displayed on the liquid crystal monitor 1025.

The detainment snare treatment instrument 1120A or the clip treatment instrument 1120B is a treatment instrument to be detained in a living body for a short term for arrest of hemorrhage and the like, so that, by providing a source coil portion 1160, a position of the treated region and an endoscope image can be recorded simultaneously with the inspection and treatment with the electronic endoscope 1006. As a result, the inspection can be effectively performed. Furthermore, at the time of re-inspection, or later, a presence or absence (excreted or remaining) of the clip or the snare can be confirmed without the X-ray fluoroscopy.

In addition, as shown in FIG. 56, the source coil portion 1160 can be applied to a drainage tube 1300 which is a treatment instrument to be detained for a long term.

Also, an RFID tag may be provided in the vicinity of the source coil portion 1160 of the treatment instrument for detainment, and in this case, information on what kind of treatment instrument is used and when it is used is recorded in this RFID tag. Accordingly, the information recorded in the RFID tag can be read out by finding out the position of the treatment instrument in the body cavity with the source coil portion 1160.

In addition, by forming the source coil 1140 at a part of the coil sheath 1151, as shown in FIG 57, treatment position by the treatment instrument can be detected without increasing the number of components. Moreover, though the insertion shape is estimated by disposing the probe 1015 in the electronic endoscope 1006 in the present example, the insertion shape of the electronic endoscope 1006 may be detected by disposing in the forceps channel 1122 the coil sheath 1151 at a part of which a plurality of source coils 1014i are formed, as shown in FIG. 58.

The present invention is not limited to the above described embodiment, and various changes and modifications can be made without departing from the scope of the present invention.

## Claims

1. A surgery assisting apparatus (1), comprising:
a probe (15) including one of either a magnetic-field generating element (14i, 140, 141) or a magnetic-field detecting element (22j) disposed in plural numbers inside an insertion portion to be inserted into a body of a subject;
a treatment instrument (100) including the one of the either elements (14i, 140, 141, 22j) disposed in plural numbers near a treatment portion (110) for performing treatment on a target region of the subject by applying energy to the target region; and
detecting means (21) for detecting respective positions of the one of the either elements (14i, 140, 141, 22j) disposed in the probe (15) and the one of the either elements (14i, 140, 141, 22j) disposed in the treatment instrument (100) using a position of the other of the either elements (14i, 140, 141, 22j) as a benchmark, by disposing the other of the either magnetic-field generating element (14i, 140, 141) or the magnetic-field detecting element (22j) outside the subject, wherein
the detecting means (21) is adapted to detect proximity of the treatment instrument (100) to the target region based on respective positions of the one of the either elements (14i, 140, 141, 22j) disposed in plural numbers in the treatment instrument (100), to calculate the shortest distance (L) between the treatment portion (110) and the probe (15), and to cause warning information (202) to be displayed on display means (25) when the shortest distance (L) is less than a first distance (L0)
**characterized in that** the detecting means (21) is adapted to stop a supply of energy to the treatment instrument (100) when the shortest distance (L) is less than a second distance (Lmin) shorter than the first distance (L0).

2. The surgery assisting apparatus (1) according to claim 1, wherein the detecting means (21) is adapted to cause the warning information (202) and distance information (201) indicating the shortest distance (L) to be displayed on the display means (25).

3. The surgery assisting apparatus (1) according to claim 1 or 2, further comprising:
shape image generating means for generating a shape image of the probe (15) and a distal end image of the treatment portion (110), based on the positions of the respective elements (14i, 140, 141, 22j) obtained by the detecting means (21), and causing the generated shape image of the probe (15) and distal end image of the treatment portion (110) to be displayed on the display means (25).

4. The surgery assisting apparatus (1) according to claim 3, further comprising an endoscope apparatus (1002) for picking up an image of the target region of the subject, wherein the detecting means (21) is adapted to extract an image part of the treatment instrument (100) from an endoscope image of the target region picked up by the endoscope apparatus (1002), and correct orientations of the shape image of the probe (15) and the distal end image of the treatment portion (110) such that the distal end image of the treatment portion (110) coincides with an image position of the extracted image part.

5. The surgery assisting apparatus (1) according to any one of claims 1 to 4, wherein the probe (15) is a guide wire (15a).

6. The surgery assisting apparatus (1) according to any one of claims 1 to 4, wherein the probe (15) is a catheter (160).

7. The surgery assisting apparatus (1) according to any one of claims 1 to 4, wherein the probe (15) is an endoscope (1006).

## Patentansprüche

1. Vorrichtung zur Unterstützung von Chirurgie (1) aufweisend:
eine Sonde (15) enthaltend ein Magnetfelderzeugungselement (14i, 140, 141) oder ein Magnetfelderfassungselement (22j), das in einer Mehrzahl in einem Einführungsteil zum Einführen in einen Körper eines Subjekts angeordnet ist;
ein Behandlungsinstrument (100) enthaltend das eine der beiden Elemente (14i, 140, 141, 22j), das in einer Mehrzahl in der Nähe eines Behandlungsteils (110) zum Ausführen einer Behandlung in einem Zielbereich des Subjekts durch Anlegen von Energie an dem Zielbereich angeordnet ist; und
Erfassungsmittel (21) zum Erfassen jeweiliger Positionen des einen der beiden Elemente (14i, 140, 141, 22j), die in der Sonde (15) angeordnet sind, und dem einen der beiden Elemente (14i, 140, 141, 22j), das in dem Behandlungsinstrument (100) angeordnet ist, unter Verwendung einer Position des anderen der beiden Elemente (14i, 140, 141, 22j) als ein Bezugswert durch Anordnen des anderen entweder des Magnetfelderzeugungselements (14i, 140, 141) oder des Magnetfelderfassungselements (22j) außerhalb des Subjekts, wobei
die Erfassungsmittel (21) dazu angepasst sind, eine Nähe des Behandlungsinstruments (100) bei dem Zielgebiet basierend auf jeweiligen Positionen des einen der beiden Elemente (14i, 140, 141, 22j), die in einer Mehrzahl in dem Behandlungsinstrument (100) angeordnet sind, zu erfassen, den kürzesten Abstand (L) zwischen dem Behandlungsteil (110) und der Sonde (15) zu berechnen und zu verursachen, dass Warnungsinformation (202) auf Anzeigemitteln (25) angezeigt wird, wenn der kürzeste Abstand (L) kleiner als ein erster Abstand (L0) ist,
**dadurch gekennzeichnet, dass** die Erfassungsmittel (21) dazu eingerichtet sind, eine Zuführung von Energie zu dem Behandlungsinstrument (100) zu stoppen, wenn der geringste Abstand (L) kleiner als ein zweiter Abstand (Lmin) ist, der geringer als der erste Abstand (L0) ist.

2. Vorrichtung zur Unterstützung von Chirurgie (1) nach Anspruch 1, bei der die Erfassungsmittel (21) dazu eingerichtet sind, zu veranlassen, dass die Warnungsinformation (202) und Abstandsinformation (201), die den geringsten Abstand (L) anzeigt, auf den Anzeigemitteln (25) angezeigt werden.

3. Vorrichtung zur Unterstützung von Chirurgie (1) nach Anspruch 1 oder 2, ferner aufweisend:
Formbilderzeugungsmittel zum Erzeugen eines Formbildes der Sonde (15) und eines Distalendbildes des Behandlungsteils (110) basierend auf den Positionen der jeweiligen Elemente (14i, 140, 141, 22j), die durch die Erfassungsmittel (21) erlangt wurden, und veranlassen, dass das erzeugte Formbild der Sonde (15) und das Distalendbild des Behandlungsteils (110) auf den Anzeigemitteln (25) angezeigt werden.

4. Vorrichtung zur Unterstützung von Chirurgie (1) nach Anspruch 3, ferner aufweisend eine Endoskopvorrichtung (1002) zum Aufnehmen eines Bildes des Zielbereichs des Subjekts, wobei die Erfassungsmittel (21) dazu eingerichtet sind, ein Bildteil des Behandlungsinstruments (100) von einem Endoskopbild des Zielbereichs, das durch die Endoskopvorrichtung (1002) aufgenommen wurde, zu extrahieren, und Orientierungen des Formbildes der Sonde (15) und des Distalendbildes des Behandlungsteils (110) so zu korrigieren, dass das Distalendbild des Behandlungsteils (110) mit einer Bildposition des extrahierten Bildteils übereinstimmt.

5. Vorrichtung zur Unterstützung von Chirurgie (1) nach einem der Ansprüche 1 bis 4, bei der die Sonde (15) ein Führungsdraht (15a) ist.

6. Vorrichtung zur Unterstützung von Chirurgie (1) nach einem der Ansprüche 1 bis 4, bei der die Sonde (15) ein Katheter (160) ist.

7. Vorrichtung zur Unterstützung von Chirurgie (1) nach einem der Ansprüche 1 bis 4, bei der die Sonde (15) ein Endoskop (1006) ist.

## Revendications

1. Appareil (1) d'assistance chirurgicale, comprenant :
une sonde (15) incluant un parmi soit un élément (14i, 140, 141) de génération de champ magnétique, soit un élément (22j) de détection de champ magnétique disposé en nombres multiples à l'intérieur d'une partie d'insertion destinée à être insérée dans un corps d'un sujet ;
un instrument (100) de traitement incluant celui parmi l'un ou l'autre élément (14i, 140, 141, 22j) disposé en nombres multiples à côté d'une partie (110) de traitement pour exécuter un traitement sur une région cible du sujet en appliquant une énergie à la région cible ; et
un moyen (21) de détection pour détecter des positions respectives de celui parmi l'un ou l'autre élément (14i, 140, 141, 22j) disposé dans la sonde (15) et celui parmi l'un ou l'autre élément (14i, 140, 141, 22j) disposé dans l'instrument (100) de traitement en utilisant une position de l'autre parmi l'un ou l'autre élément (14i, 140, 141, 22j) comme un repère, en disposant l'autre parmi soit l'élément (14i, 140, 141) de génération de champ magnétique, soit l'élément (22j) de détection de champ magnétique à l'extérieur du sujet, dans lequel
le moyen (21) de détection est adapté à détecter une proximité de l'instrument (100) de traitement par rapport à la région cible sur la base de positions respectives de celui parmi l'un ou l'autre élément (14i, 140, 141, 22j) disposé en nombres multiples dans l'instrument (100) de traitement, à calculer la distance la plus courte (L) entre la partie (110) de traitement et la sonde (15), et à faire en sorte qu'une information (202) d'avertissement soit affichée sur un moyen (25) d'affichage lorsque la distance la plus courte (L) est inférieure à une première distance (L0),
**caractérisé en ce que** le moyen (21) de détection est adapté à arrêter une alimentation en énergie de l'instrument (100) de traitement lorsque la distance la plus courte (L) est inférieure à une deuxième distance (Lmin) plus courte que la première distance (L0).

2. Appareil (1) d'assistance chirurgicale selon la revendication 1, dans lequel le moyen (21) de détection est adapté à faire en sorte que l'information (202) d'avertissement et une information (201) de distance indiquant la distance la plus courte (L) soient affichées sur le moyen (25) d'affichage.

3. Appareil (1) d'assistance chirurgicale selon la revendication 1 ou 2, comprenant en outre :
un moyen de génération d'image de forme pour générer une image de forme de la sonde (15) et une image d'extrémité distale de la partie (110) de traitement, sur la base des positions des éléments (14i, 140, 141, 22j) respectifs obtenues par le moyen (21) de détection, et faire en sorte que l'image de forme de la sonde (15) et l'image d'extrémité distale de la partie (110) de traitement générées soient affichées sur le moyen (25) d'affichage.

4. Appareil (1) d'assistance chirurgicale selon la revendication 3, comprenant en outre un appareil endoscopique (1002) pour capturer une image de la région cible du sujet, dans lequel le moyen (21) de détection est adapté à extraire une partie d'image de l'instrument (100) de traitement à partir d'une image endoscopique de la région cible capturée par l'appareil endoscopique (1002), et corriger des orientations de l'image de forme de la sonde (15) et de l'image d'extrémité distale de la partie (110) de traitement de façon à ce que l'image d'extrémité distale de la partie (110) de traitement coïncide avec une position d'image de la partie d'image extraite.

5. Appareil (1) d'assistance chirurgicale selon l'une quelconque des revendications 1 à 4, dans lequel la sonde (15) est un fil-guide (15a).

6. Appareil (1) d'assistance chirurgicale selon l'une quelconque des revendications 1 à 4, dans lequel la sonde (15) est un cathéter (160).

7. Appareil (1) d'assistance chirurgicale selon l'une quelconque des revendications 1 à 4, dans lequel la sonde (15) est un endoscope (1006).
